# EUROPÄISCHE PATENTSCHRIFT

(11) **EP 1 177 199 B1**
(45) Veröffentlichungstag und Bekanntmachung des Hinweises auf die Patenterteilung: **11.09.2002**
(21) Anmeldenummer: 00929379.6
(22) Anmeldetag: 18.04.2000
(51) Int. Cl.: C07F 9/141, C07F 9/6574, C07C 211/63, B01J 31/02

(54) **IONISCHE FLÜSSIGKEITEN, IHRE HERSTELLUNG UND IHRE VERWENDUNG**
IONIC LIQUIDS AND PRODUCTION AND USE THEREOF
LIQUIDES IONIQUES, LEUR PRODUCTION ET LEUR UTILISATION

(30) Priorität: 29.04.1999 DE 19919494
(43) Veröffentlichungstag der Anmeldung: 06.02.2002
(73) Patentinhaber: Celanese Chemicals Europe GmbH, 60439 Frankfurt am Main (DE)
(72) Erfinder: BAHRMANN, Helmut, D-46499 Hamminkeln (DE); BOHNEN, Hans, D-47441 Moers (DE)
(86) Internationale Anmeldenummer: EP0003499
(87) Internationale Veröffentlichungsnummer: WO00066597

(56) Entgegenhaltungen:
- EP-A- 0 003 554
- EP-A- 0 776 880
- GB-A- 2 337 754
- US-A- 5 892 124
- BÖHM ET AL.: "Nonaqueous ionic liquids: superior reaction media for the catalytic heck-vinylation of chloroarenes" CHEMISTRY A EUROPEAN JOURNAL, Bd. 6, Nr. 6, März 2000 (2000-03), Seiten 1017-1025, XP002901206
- POOLE S. ET AL: "Chromatographic and spectroscopic studies of the solvent properties of a new series of roomtemperature liquid tetraalkylammonium sulfonates" ANALYTICA CHIMICA ACTA, Bd. 218, 1989, Seiten 241-264, XP002901207

## Beschreibung

Die vorliegende Erfindung betrifft neue ionische Flüssigkeiten, die als Medien für die Durchführung chemischer Reaktionen, insbesondere Reaktionen, die in Gegenwart von Katalysatoren ablaufen, eingesetzt werden. Die neuen ionischen Flüssigkeiten sind Salze sulfonierter oder carboxylierter Triester der phosphorigen Säure als anionischem, und Ammonium-Ionen, die durch organische Reste substituiert sein können, als kationischem Bestandteil.

lonische Systeme, die bei niedriger Temperatur schmelzen und im Bereich von etwa Raumtemperatur bis zu einigen hundert Grad Celsius flüssig sind, können als Reaktionsmedien für ein breites Spektrum chemischer Prozesse eingesetzt werden. Die ionischen Flüssigkeiten übernehmen in dieser Verwendungsart häufig eine doppelte Aufgabe: sie dienen nicht nur als Lösungsmittel für die Reaktanten, sondern gleichzeitig auch als Katalysatoren oder als Katalysatorkomponenten für die Umsetzung der Reaktionspartner zum gewünschten Produkt. Die Vorteile einer solchen Umsetzung in homogener Phase sind bekannt. Man erzielt häufig hohe Reaktionsgeschwindigkeiten und die Chemo-, Regio-, Stereo- und Enantioselektivität des reaktiven Geschehens läßt sich oftmals einfach und präzise steuern. In Sonderfällen ist das Reaktionsprodukt im Reaktionsmedium nicht löslich. Dann ergibt sich als weiterer Vorteil die leichte Trennung von Produkt, Katalysator und Ausgangsstoffen.

In einer anderen Variante der chemischen Reaktionsführung setzt man ionische Flüssigkeiten ein, die lediglich als Lösungsmittel für den Katalysator dienen, die mit den Reaktanten und dem Reaktionsprodukt jedoch nicht mischbar sind. Die Umsetzung der Reaktionspartner erfolgt in diesem Fall an der Phasengrenzfläche zur Katalysatorlösung und das Reaktionsprodukt bildet eine eigene, vom Katalysator getrennte Phase. Dieser Prozeß, der als Umsetzung in zwei heterogenen flüssigen Phasen beschrieben werden kann, erweist sich immer dann als zweckmäßig, wenn das Reaktionsprodukt schnell aus dem Reaktionsgemisch entfernt werden muß, um es nicht Folgereaktionen auszusetzen. Überdies lassen sich Katalysator und Reaktionsprodukt unter schonenden Bedingungen, insbesondere unter Ausschluß thermischer Behandlungsmethoden, die zu einer Schädigung der Bestandteile des Reaktionsgemisches führen können, voneinander trennen. Auch bei katalytischen Reaktionen, die in heterogenen Systemen ablaufen, kann die ionische Flüssigkeit nicht nur als Lösungsmittel für den Katalysator wirken, sondern selbst Bestandteil des Katalysators sein.

Weitere Vorzüge der ionischen Flüssigkeiten sind ihre chemische und thermische Stabilität, die ihnen weite Anwendungsgebiete eröffnen. Wegen ihres nicht meßbaren Dampfdruckes emittieren sie keine Dämpfe, sie tragen infolgedessen nicht zur Luftverschmutzung bei und sind, verglichen mit herkömmlichen, als Reaktionsmedien verwendeten Lösungsmitteln, bemerkenswert umweltverträglich. Ionische Flüssigkeiten finden aufgrund der geschilderten mannigfachen Vorteile zunehmendes Interesse als reaktive Komponente oder als Reaktionshilfsmittel bei zahlreichen industriell durchgeführen Synthesen.

Nach CHEMTECH, September 1995, Seiten 26ff, verwendet man bei Raumtemperatur flüssige, ionische Flüssigkeiten, z.B. eine Mischung aus 1,3-Dialkylimidazoliumchlorid, vorzugsweise 1-n-Butyl-3-methylimidazoliumchlorid (abgekürzt [BMI]⁺[Cl]⁻) und Aluminiumchlorid und/oder Ethylaluminiumchlorid, als nichtwäßrige Lösungsmittel für Katalysatoren. Als Beispiel einer Reaktion, bei der solche Katalysatorlösungen eingesetzt werden, führt die Veröffentlichung die Dimerisierung von Olefinen in Gegenwart von Nickel-Komplexverbindungen als Katalysator an, z.B. die Dimerisierung von Propen zu isomeren Hexenen und die Dimerisierung von Buten zu Iso-Octenen. Das Reaktionsgemisch bildet zwei Phasen, von denen das Reaktionsprodukt die obere bildet und die untere aus der Katalysatoriösung besteht. Nach Trennung der Phasen kann die Katalysatorlösung in den Prozeß zurückgeführt werden.

Aus Am.Chem.Soc., Div. Pet. Chem. (1992), 37, Seiten 370 ff ist bekannt, Propen in Gegenwart einer Lösung von NiCl₂ · (PR₃)₂, (R=i-C₃H₇), in einer Mischung aus [BMI]⁺[Cl]⁻ und AlCl₃ als ionischer Flüssigkeit zu dimerisieren.

Die Verwendung von niedrig schmelzenden Phosphoniumsalzen, z.B. Tetrabutylphosphoniumbromid als Lösungsmittel in Hydroformylierungsreaktionen wird im Journal of Molecular Catalysis, 47 (1988), Seiten 99ff beschrieben. So ergibt die Hydroformylierung von Octen-1 mit Rutheniumcarbonyl-Komplexverbindungen in Gegenwart stickstoff- und phosphorhaltiger Liganden, z.B. 2,2'-Dipyridyl oder 1,2-Bis(diphenylphosphino)-ethan bei 120 bis 180°C ein Gemisch aus n-Nonanol und n-Nonanal mit einem Anteil von bis zu 69 Gew.-% des Alkohols, bezogen auf das Reaktionsgemisch. Zur Isolierung des gewünschten n-Nonanals ist daher eine aufwendige Destillation erforderlich.

Die europäische Patentanmeldung EP-A-0 776 880 lehrt die Hydroformylierung von Olefinen in Gegenwart von quaternären Ammonium- und/oder Phosphoniumsalzen als Lösungsmittel für den Katalysator. Bevorzugt werden Salze, die [BMI]⁺ als Kation enthalten. Auch Salze quatemärer Diamine mit Kationen der allgemeinen Formel

R¹R²N^{⊕}=CR³-R⁵-R³C=N^{⊕}R¹R²

wobei in dieser Veröffentlichung R¹, R², R³ gleich oder verschieden sind und Wasserstoff oder einen Kohlenwasserstoffrest mit 1 bis 12 Kohlenstoffatomen bedeuten und R⁵ ein Alkylenrest, z.B. Methylen, Ethylen oder Propylen oder ein Phenylenrest ist, werden verwendet. Geeignete Anionen sind beispielsweise das Hexafluorophosphat-, Hexafluoroantimonat-, Tetrachloroaluminat- oder das Tetrafluoroboration. Diese quaternären Ammoniumund/oder Phosphoniumsalze sind bereits unterhalb von 90°C, vorzugsweise unterhalb von 85°C und besonders bevorzugt unterhalb von 50°C, flüssig.

Der in diesen Salzen gelöste Hydroformylierungskatalysator enthält als aktives Metall Kobalt, Rhodium, Iridium, Ruthenium, Palladium oder Platin und als Ligand ein tertiäres Phosphin oder ein tertiäres sulfoniertes Phosphin, ein tertiäres Arsin, ein tertiäres Stilbin oder ein Phosphit. Das Molverhältnis von Ligand zu Metall beträgt 9,5.

Die katalytisch wirksamen Metalle werden als Verbindungen, Rhodium z.B. in Form von Rhodiumacetylacetonatdicarbonyl oder Rhodiumcarbonyl Rh₆(CO)₁₆ eingesetzt. Aus ihnen bildet sich unter den Reaktionsbedingungen der Hydroformylierungskatalysator. Besonders bevorzugt wird die Hydroformylierungsreaktion zwischen 30 und 90°C durchgeführt.

Auch nach Angew. Chem. 1995, 107, Nr. 23/24, Seiten 2941 ff lassen sich Hydroformylierungsreaktionen unter Verwendung bei Raumtemperatur flüssiger 1,3-Dialkylimidazoliumsalze als katalysatorhaltiges, nicht mit dem organischen Reaktionsgemisch mischbares Lösungsmittel durchführen. Hierzu wird Rhodiumdicarbonylacetylacetonat als Katalysatorvorstufe zu einer Lösung von Triphenylphospin in [BMI]^{⊕}-[PF6]^{⊖} gegeben, wobei das Molverhältnis Phosphor(III) zu Rhodium von 3 bis 10 variieren kann. Der Katalysator wird mit Synthesegas (Volumenverhältnis von Wasserstoff zu Kohlenmonoxid gleich 1 : 1) präformiert. Anschließend setzt man n-Penten-1 mit Synthesegas gleicher Zusammensetzung bei einer Temperatur von 80°C um. Auch in diesem Falle läßt sich die organische Produktphase in einfacher Weise von der katalysatorhaltigen, nichtwäßrigen ionischen Flüssigkeit durch Dekantieren abtrennen.

Die bekannten Hydroformylierungsverfahren verwenden als ionische Flüssigkeiten zum Lösen des Katalysators Salze, deren Anionen gegenüber dem katalytisch wirksamen Metall keine Ligandeigenschaften haben und daher nicht zur Bildung von Komplexverbindungen befähigt sind. Im günstigen Fall liegen sie im Reaktionsgemisch als Inerte vor und greifen nicht in das Reaktionsgeschehen ein. Es ist jedoch nicht auszuschließen, daß sie den Reaktionsablauf negativ beeinflussen, z.B. die Reaktionsgeschwindigkeit mindern oder die Selektivität herabsetzen.

Weiterhin folgt aus dem Stand der Technik (vgl. Angew. Chem. 1995, 107, S. 2941 und EP-A-0 776 880), daß sich das Molverhältnis Ligand/Metall, z.B. Phosphor/Rhodium, im Bereich von 3 bis 10 bewegt. Höhere Molverhältnisse werden offensichtlich als ungeeignet angesehen, obgleich eine Vergrößerung des Ligandanteils, bezogen auf das Metall, die Stabilität der katalytisch wirksamen Komplexverbindung verbessern sollte. Möglicherweise ist die Löslichkeit der als Liganden wirkenden Verbindungen in den bisher gebräuchlichen ionischen Flüssigkeiten begrenzt, so daß sie sich bei Überschreiten einer maximalen Konzentration aus der Lösung abscheiden und aus der Katalysatorphase ausgetragen werden.

In der EP-A2-0 353 770 wird die Durchführung katalytischer Prozesse in homogener Phase und in Gegenwart von Katalysatorsystemen beschrieben, die aus Komplexverbindungen von Übergangsmetallen der VIII. Gruppe des Periodensystems und ionischen Phosphiten als Liganden bestehen. Unter dem Begriff "ionische Phosphite" werden Salze von Estern der phosphorigen Säure verstanden, deren Alkoholkomponente durch Sulfonat- oder Carboxylatreste substituiert und daher zur Salzbildung befähigt sind. Üblicherweise sind die ionischen Phosphite im Überschuß vorhanden, d.h. neben den Metall-Komplexverbindungen liegen freie Liganden vor, die keine Bindung mit dem Übergangsmetall eingegangen sind. Das Katalysatorsystem, also Übergangsmetall und ionisches Phosphit, wird in einem organischen Lösungsmittel, z.B. aliphatischen Aldehyden, mit 3 bis 6 Kohlenstoffatomen im Molekül, gelöst, eingesetzt, das Phosphit selbst ist nicht Lösungsmittel. Zur Verbesserung der Löslichkeit des Katalysators fügt man dem Lösungsmittel häufig noch einen Lösungsvermittler zu. Anwendung findet diese spezielle Ausgestaltung eines katalytischen Verfahrens z.B. bei der Hydroformylierung von Olefinen. Nach den Angaben in der EP-A2-0 353 770 zeichnen sich die mit den beschriebenen ionischen Phosphitliganden gebildeten Katalysatoren durch hohe Aktivität aus. Die Umsetzung von Olefinen mit CO und H₂ führt bevorzugt zu unverzweigten Aldehyden und bei Abtrennung der Reaktionsprodukte aus dem Reaktionsgemisch durch Destillation erweist sich die geringe Flüchtigkeit der ionischen Phosphite als vorteilhaft.

Die bekannten Verfahren zur Durchführung katalytischer Reaktionen in ionischen Flüssigkeiten befriedigen noch nicht in jeder Beziehung. So erfüllen der Umsatz der Einsatzstoffe und die Selektivität hinsichtlich der Produkte nicht immer die von technisch ausgeübten Prozessen verlangte Wirtschaftlichkeit. Häufig ist auch der Austrag des Katalysatormetalls, oftmals ein Edelmetall, zusammen mit dem Reaktionsprodukt aus dem Reaktionsgemisch nicht tolerierbar. Bei Mischbarkeit von Reaktionsmedium und Endstoffen sind energieintensive und, im Falle thermisch labiler Produkte, technisch aufwendige Trennungsoperationen durchzuführen.

Der Einsatz von ionischen Phosphiten als Liganden für Katalysatoren auf Basis von Metallkomplex-Verbindungen bedient sich, wie z.B. EP-A2-0 353 770 zeigt, herkömmlicher Reaktionsmedien - organische Lösungsmittel - und findet seine Grenzen in der beschränkten Löslichkeit dieser Substanzklasse in organischen Medien.

Es bestand daher die Aufgabe, ionische Flüssigkeiten bereitzustellen, die breite Anwendung als Lösungsmittel für Katalysatoren finden können, gegebenenfalls selbst Katalysatoren oder Komponenten von Katalysatorsystemen sind und deren Lösungsvermögen für anorganische und organische Substanzen, Ausgangsstoffe und Reaktionsprodukte den jeweiligen Anforderungen entsprechend eingestellt werden kann. Vorzugsweise sollen sie zumindest mit den Reaktionsprodukten keine Mischung eingehen, so daß die Möglichkeit besteht, Einsatzstoffe und Katalysator von den Endstoffen einfach zu trennen.

Die vorstehend skizzierte Aufgabe wird gelöst durch nichtwäßrige ionische Flüssigkeiten der allgemeinen Formel (Q⁺)ₐA^{a-}, in der Q⁺ ein einfach geladenes, gegebenenfalls durch organische Reste substituiertes Ammonium-Kation oder das Äquivalent eines mehrfach geladenen, gegebenenfalls durch organische Reste substituierten Ammonium-Kations ist, A^{a-} für das Anion eines sulfonierten oder carboxylierten Triesters der phosphorigen Säure steht und a eine ganze Zahl und mindestens gleich 1 ist.

Überraschenderweise wurde gefunden, daß sich die erfindungsgemäßen nichtwäßrigen ionischen Lösungsmittel hervorragend als Reaktionsmedium für Reaktionen insbesondere organischer Stoffe mit organischen oder anorganischen Reaktanten eignen. Sie sind Solventien für eine Vielzahl in chemischen Synthesen eingesetzten Katalysatoren und können aufgrund des Vorliegens dreibindigen Phosphors selbst Katalysatoren bzw. Bestandteile von Katalysatoren, z.B. von katalytisch wirkenden Metallkomplex-Verbindungen, sein. Durch Variation der Struktur des Esteranions und des Ammoniumkations, durch Mischung unterschiedlicher Verbindungen der erfindungsgemäßen Art untereinander, gegebenenfalls auch mit Estersalzen, deren Kation kein Ammonium-Ion ist, lassen sich ionische Lösungsmittel bereiten, die individuellen Erfordernissen, z.B. hinsichtlich ihres thermischen Verhaltens oder ihres Lösungsvermögens für bestimmte Stoffe, anorganische wie organische, angepaßt sind.

Die gemäß der Erfindung eingesetzten Ammoniumsalze sulfonierter oder carboxylierter Phosphorigsäuretriester lassen sich formal von der phosphorigen Säure durch Veresterung mit den Ammoniumsalzen von Hydroxysulfonsäuren bzw. Hydroxycarbonsäuren der allgemeinen Formel

(Qac)_{b}-Y-(OH)_{c} (1)

ableiten, in der ac einen aciden Rest, nämlich den Sulfonsäurerest -SO₃⁻ bzw. den Carbonsäurerest -COO⁻ und Q, wie bereits angegeben, ein einfach geladenes, gegebenenfalls durch organische Reste substituiertes Ammonium-Kation oder das Äquivalent eines mehrfach geladenen, gegebenenfalls durch organische Reste substituiertes Ammonium-Kations bedeuten.

Weiterhin steht in der allgemeinen Formel (1) Y für einen organischen Rest. Dementsprechend fallen unter diese Formel sulfonierte oder carboxylierte Hydroxyverbindungen, die sich von aliphatischen, cycloaliphatischen, aromatischen und heterocyclischen Grundstrukturen ableiten. Die aliphatischen Verbindungen können linear oder verzweigt und, wie die cycloaliphatischen Verbindungen, gesättigt oder ungesättigt sein. Zu den cycloaliphatischen und den aromatischen Verbindungen zählen sowohl einkernige als auch mehrkemige Strukturen. Ebenso gehören zu den Hydroxysäuren der erfindungsgemäß eingesetzten Phosphite aliphatisch-aromatische wie auch aromatisch-aliphatische Verbindungen. Als heterocyclische Verbindungen kommen gesättigte oder ungesättigte Ringsysteme mit Stickstoff, Sauerstoff oder Schwefel als Heteroatom in Betracht. Im Molekül können auch zwei oder mehr gleiche oder verschiedene Heteroatome enthalten sein. Überdies kann der Heterocyclus durch Alkylreste oder Arylreste substituiert oder mit weiteren Ringsystemen, aliphatischen, aromatischen oder heterocyclischen, kondensiert sein. Alle Verbindungen können noch weitere Substituenten tragen, von denen der Fachmann weiß, daß sie sich bei ihrer speziellen Anwendung als ionische Flüssigkeit inert verhalten.

Insbesondere steht Y in der obigen Formel (1) für lineare oder verzweigte, gesättigte aliphatische Reste mit insgesamt 1 bis 20 Kohlenstoffatomen, die durch Hydroxy- oder durch Alkoxyreste mit 1 bis 10 Kohlenstoffatomen substituiert sein können. Y bedeutet weiterhin vorzugsweise gesättigte oder ungesättigte ein- oder mehrkemige cycloaliphatische Reste mit 5 bis 14 Kohlenstoffatomen im Ring oder den Ringen und ein- oder mehrkemige aromatische Reste mit 6 bis 14 Kohlenstoffatomen im Ring oder den Ringen. Sowohl die cycloaliphatischen als auch die aromatischen Reste können neben Sulfonsäure- oder Carbonsäurereste noch weitere Substituenten enthalten, nämlich Alkylreste mit 1 bis 20 Kohlenstoffatomen, Aryl-, Alkylaryl- oder Aralkylreste mit 6 bis 30 Kohlenstoffatomen und Cycloalkylreste mit 5 bis 14 Kohlenstoffatomen, ferner Hydroxygruppen sowie Alkoxyreste mit 1 bis 10 Kohlenstoffatomen. Die aromatischen Reste leiten sich vorzugsweise vom Benzol, vom Biphenyl, vom Naphthalin und vom Binaphthyl ab. Als Arylalkylrest hat sich vor allem der leicht zugängliche, gegebenenfalls substituierte Benzylrest bewährt. Alkylarylreste gehen bevorzugt auf Toluol, Ethylbenzol und die isomeren Xylole zurück. Unter den Heterocyclen sind Reste Stickstoff enthaltender, gesättigter oder ungesättigter Fünf- oder Sechsringe von Bedeutung, insbesondere Pyridin. b und c schließlich sind jeweils ganze Zahlen und haben jeweils mindestens den Wert 1, c ist insbesondere 1 oder 2.

Zu den sulfonierten oder carboxylierten Estem der phosphorigen Säure gemäß der Erfindung gehören insbesondere Verbindungen der allgemeinen Formel (2)

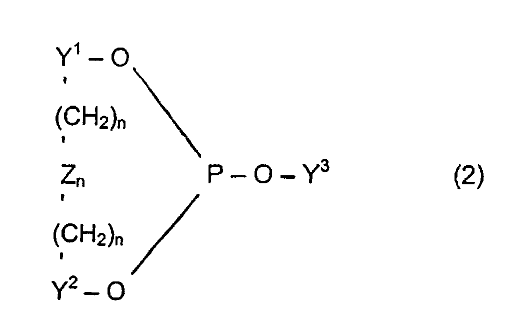

in der Y¹, Y² und Y³ gleich oder verschieden sind, einen organischen Rest bedeuten und die für Y unter Formel (1) genannte Bedeutung haben. Z ist eine zweiwertige Brückengruppe und steht für -CR¹R²-, wobei R¹ und R² unabhängig voneinander Wasserstoff oder Alkylreste mit 1 bis 12 Kohlenstoffatomen sind. Z steht weiterhin für -O-, -S-, -CO-, -CH₂-CO-CH₂-, n ist gleich oder verschieden und entspricht 0 oder 1 und im Falle Z gleich - CR¹R²- 1, 2 oder 3. Wenn n den Wert 0 hat, können die Reste Y¹, Y² und Y³ selbständig sein. Zwei benachbarte Reste Y¹ und Y², Y² und Y³ oder Y¹ und Y³ können aber auch miteinander verbunden sein und z.B. einen bivalenten Rest bilden. Ist Y¹ und Y² jeweils ein sich vom Benzol ableitender Rest, so können diese beiden benachbarten Reste z.B. durch eine Einfachbindung zu einem bivalenten Biphenylrest verknüpft sein. Sofern benachbarte Reste Y¹, Y², Y³ cycloaliphatische oder aromatische Strukturen bedeuten, können sie im Falle n = 0 auch linear ankondensiert (anneliert) sein. Es resultieren dann z.B. bivalente, gegebenenfalls substituierte cycloaliphatische, aromatische oder cycloaliphatisch-aromatische Reste, z.B. zweibindige Dicyclodecylenoder Tricyclotetradecylenreste oder zweibindige Naphthylen- oder Anthracylenreste. Weiterhin enthalten die Ester der allgemeinen Formel (2) mindestens einen ac⁻-Rest, also mindestens eine Sulfonsäuregruppe oder eine Carboxylgruppe.

In den Verbindungen der allgemeinen Formel (2) ist Y¹, Y², Y³ vorzugsweise ein vom Benzol, vom Naphthalin, vom Biphenyl oder vom Binaphthyl abgeleiteter Rest, der jeweils durch einen oder mehrere Alkylreste mit 1 bis 20 Kohlenstoffatomen, durch einen oder mehrere Aryl-, Aralkyl-, Alkylarylreste mit 6 bis 30 Kohlenstoffatomen und/oder durch einen oder mehrere Cycloalkylreste mit 5 bis 14 Kohlenstoffatomen, durch Hydroxygruppen und/oder Alkoxyreste mit 1 bis 10 Kohlenstoffatomen und/oder durch einen oder mehrere Säurereste (-ac⁻) substituiert sein kann. Z steht insbesondere für den Rest -CH₂-, für -O-, -CO- und -CH₂-O-CH₂-.

Zu den der allgemeinen Formel (2) entsprechenden Verbindungen gehören Sulfonate oder Carboxylate von Trialkylphosphiten wie Trimethylphosphit, Triethylphosphit, Butyldiethylphosphit, Tri-n-propylphosphit, Tri-n-butylphosphit, Tri-2-ethylhexylphosphit, Tri-n-octylphosphit, Tri-n-dodecylphosphit, von Dialkylarylphosphiten wie Dimethylphenylphosphit, Diethylphenytphosphit, von Alkyldiarylphosphiten wie Methyldiphenylphosphit, Ethyldiphenylphosphit und von Triarylphosphiten wie Triphenylphosphit, Phenylbiphenylenphosphit und Trinaphthylphosphit.

Eine weitere Gruppe bedeutsamer sulfonierter oder carboxylierter Ester der phosphorigen Säure entsprechend der Erfindung sind Polyphosphite der allgemeinen Formel (3)

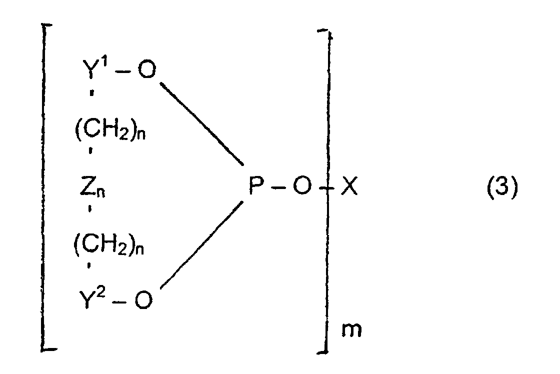

In dieser Formel sind Y¹ und Y² gleich oder verschieden und haben die unter Formel (1) für Y und unter Formel (2) für Y¹, Y² und Y³ wiedergegebenen Bedeutungen. Die Definitionen von Z und n entsprechen den Angaben unter Formel (2). X steht für eine m-wertige Brückengruppe aus der Gruppe Alkylenreste, Alkylenoxyalkylenreste, Arylenreste oder Aryl-Zₙ-arylreste. m ist eine ganze Zahl und hat einen Wert von 2 bis 6. Weiterhin enthält das Polyphosphit der allgemeinen Formel (3) mindestens einen ac⁻-Rest, also mindestens eine Sulfonat-(-SO₃⁻) oder Carboxylat-(-COO⁻)-gruppe.

X wird bevorzugt durch Alkylenreste mit 2 bis 18, insbesondere 2 bis 12, Kohlenstoffatomen und durch Arylenreste mit 6 bis 18 Kohlenstoffatomen beschrieben. In der Bedeutung Aryl-Zₙ-Aryl steht Z bevorzugt für -CH₂-, für -O-, -CO- und -CH₂-CO-CH₂-. Die durch X bezeichneten Reste können ebenfalls durch einen oder mehrere Alkyl- und/oder Alkoxyreste und/oder durch einen oder mehrere Säurereste (-ac⁻) substituiert sein.

Weitere wichtige Vertreter der erfindungsgemäßen Ester der phosphorigen Säure werden durch die nachstehende allgemeine Formel (4) wiedergegeben.

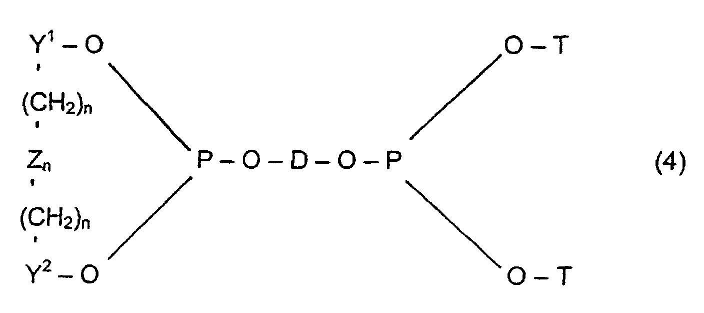

In dieser Formel sind Y¹ und Y² gleich oder verschieden und haben die unter Formel (1) für Y und unter Formel (3) für Y¹ und Y² wiedergegebene Bedeutung. Die Definitionen von Z und n entsprechen den Angaben unter Formel (2) und Formel (3). D steht für einen zweiwertigen Kohlenwasserstoffrest als Brückengruppe, nämlich einen Alkylenrest mit 1 bis 30 Kohlenstoffatomen, einen Arylen-, Alkylarylen-, Arylalkylenrest mit 6 bis 30 Kohlenstoffatomen und einen Aryl-Zₙ-aryl-Rest. T ist ein einwertiger Kohlenwasserstoffrest mit 1 bis 30 Kohlenstoffatomen und kann ein Alkyl-, Aryl-; Aralkyl-, Alkylaryl- oder Cycloalkylrest sein. Weiterhin enthält das Phosphit der allgemeinen Formel (4) mindestens einen ac⁻- Rest, also mindestens eine Sulfonat-(-SO₃⁻) oder Carboxylat (-COO⁻)-gruppe.

Die sulfonierten oder carboxylierten Ester der phosphorigen Säure kann man durch Umesterung (Alkoholyse) von Phosphorigsäureestem mit dem Salz, vorzugsweise dem Ammoniumsalz, einer Hydroxysulfonsäure oder einer Hydroxycarbonsäure erhalten. Hierzu wird das in einem organischen Lösungsmittel gelöste Salz bei 20 bis 200°C, vorzugsweise 80 bis 160°C, mit dem Phosphorigsäureester umgesetzt. Die Reaktanten werden üblicherweise in äquivalenten Mengen verwendet, wenngleich es auch möglich ist, einen der beiden Reaktionspartner im Überschuß einzusetzen. Die Reaktion wird durch Katalysatoren wie Amine, Natrium, Natriumalkoholate, Aluminiumtrichlorid, Titansäureester oder Phosphorigsäuredialkylester beschleunigt Zur Umesterung geeignete Phosphorigsäureester leiten sich von aliphatischen oder aromatischen Hydroxyverbindungen ab, vorzugsweise solchen, die 1 bis 12 Kohlenstoffatomen enthalten. Beispiele für solche Phosphite sind Trimethylphosphit, Triethylphosphit, Butyldiethylphosphit, Tri-n-propylphosphit, Tri-n-butylphosphit, Tri-2-ethylhexylphosphit, Tri-n-octylphosphit, Tri-n-dodecylphosphit, Dimethylphenylphosphit, Diethylphenylphosphit, Triphenylphospit. Bevorzugtes organisches Phosphit ist das Triphenylphosphit.

Als Kationen Q⁺ enthalten die erfindungsgemäßen nichtwäßrigen ionischen Flüssigkeiten insbesondere durch organische Reste substitutierte Ammoniumionen, nämlich Ammoniumionen, die sich von Mono- oder Diaminen ableiten. Die Ammoniumionen von Monoaminen entsprechen den allgemeinen Formeln (5) und (6)

^{⊕}NR³R⁴R⁵R⁶ (5)

und

R³R⁴N^{⊕} = CR⁵R⁶ (6)

wobei R³, R⁴, R⁵ und R⁶ gleich oder verschieden sind und Wasserstoff, insbesondere mit der Maßgabe, daß mindestens ein R³, R⁴, R⁵, R⁶ nicht Wasserstoff ist, oder einen linearen oder verzweigten, aliphatischen Kohlenwasserstoffrest mit 1 bis 20 Kohlenstoffatomen, einen cycloaliphatischen oder aromatischen Kohlenwasserstoffrest mit 6 bis 20 Kohlenstoffatomen oder einen Alkoxyrest mit 1 bis 10 Kohlenstoffatomen bedeuten. Beispiele für solche Reste sind Alkyl-, Alkenyl-, Cycloalkyl-, Aryl- Alkylaryl-, oder Aralkylreste.

Als Kationen der erfindungsgemäßen nichtwäßrigen ionischen Flüssigkeiten kommen ferner Ionen in Betracht, die sich von gesättigten oder ungesättigten cyclischen Verbindungen sowie von aromatischen Verbindungen mit jeweils einem dreibindigen N-Atom im 4- bis 10-, vorzugsweise 5- bis 6-gliedrigen heterocyclischen Ring ableiten. Solche Kationen lassen sich vereinfacht (d.h. ohne Angabe von genauer Lage und Anzahl der Doppelbindungen im Molekül) durch die nachstehenden allgemeinen Formeln (7) und (8) wiedergeben.

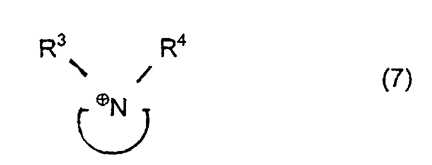

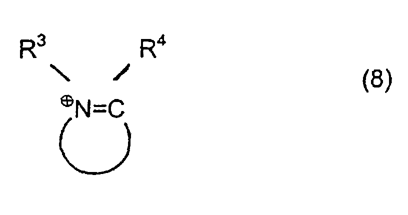

R³ und R⁴ besitzen dabei die vorgenannte Bedeutung. Beispiele für cyclische Amine der vorgenannten Art sind Pyrrolidin, Dihydropyrrol, Pyrrol, Indol, Carbazol, Piperidin, Pyridin, die isomeren Picoline und Lutidine, Chinolin und i-Chinolin.

Bevorzugte Kationen gehen auf aliphatische, cycloaliphatische oder aromatische Diamine zurück. Sie folgen den allgemeinen Formeln (9) und (10)

R³R⁴R⁵N^{⊕}-G-N^{⊕}R⁶R⁷R⁸ (9)

R³R⁴N^{⊕} = CR⁵-G-R⁵C = N^{⊕}R³R⁴ (10)

in denen R³, R⁴, R⁵, R⁶, R⁷ und R⁸ gleich oder verschieden sind und Wasserstoff, einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 30 Kohlenstoffatomen, einen Alkylarylrest mit 7 bis 40 Kohlenstoffatomen oder einen Alkoxyrest mit 1 bis 10 Kohlenstoffatomen bedeuten. G steht für einen Alkylenrest (-CHR⁹-)_{d}, wobei R⁹ Wasserstoff oder ein Kohlenwasserstoffrest mit 1 bis 5 Kohlenstoffatomen und d eine ganze Zahl von 1 bis 8, vorzugsweise 2 bis 6 ist, für einen Arylenrest mit 6 bis 30 Kohlenstoffatomen oder für einen Alkylenarylrest mit 7 bis 40 Kohlenstoffatomen. Beispiele für die durch R³, R⁴, R⁵, R⁶, R⁷ und R⁸ bezeichneten Kohlenwasserstoffreste sind Alkyl-, Alkenyl-, Cycloalkyl-, Aryl, Alkylaryl-, oder Arylalkylreste, wie Methyl, Ethyl, Propyl, i-Propyl, Butyl, sek.-Butyl, t-Butyl, Amyl, Methylen, Ethyliden, Phenyl, Benzyl. R⁹ wird beispielhaft durch den Methyl-, Ethyl-, n-Propyl-, i-Propylrest und die isomeren Butylreste beschrieben. Beispiele für G sind die Reste Methylen, Ethylen, Propylen, Butylen, 1,4-Phenylen, 1,4-Tolylen, 1,4-Xylylen, 1,1'-Biphenyl-4,4'-diyl, 1,4-Naphthylen, 1,1-Binaphthyl-2,2'-diyl.

Besonders geeignete Kationen der erfindungsgemäßen nichtwäßrigen ionischen Flüssigkeiten leiten sich von 1-Amino-3-dialkylaminopropanen der allgemeinen Formel (11)

R¹⁰R¹¹N-CH₂-CH₂-CH₂-NH₂ (11)

als Diaminen ab, in der R¹⁰ und R¹¹ gleiche oder verschiedene lineare oder verzweigte Alkylreste mit 4 bis 20 Kohlenstoffatomen sind und beispielsweise n-Butyl, n-Pentyl, n-Hexyl, n-Heptyl, i-Heptyl, n-Octyl, i-Octyl, n-Nonyl, i-Nonyl, n-Decyl, i-Decyl, n-Undecyl, i-Undecyl, n-Dodecyl oder i-Dodecyl bedeuten.

Weitere vorteilhafte Kationen der erfindungsgemäßen nichtwärigen ionischen Flüssigkeiten gehen auf folgende Amine zurück: 1-Amino-3-(di-n-heptyl)-aminopropan, 1-Amino-3-(di-i-heptyl)-aminopropan, 1-Amino-3-(di-n-octyl)-aminopropan, 1-Amino-3-(di-i-octyl)-aminopropan, 1-Amino-3-(di-n-nonyl)-aminopropan, 1-Amino-3-(di-i-nonyl)-aminopropan, 1-Amino-3-(di-n-undecyl)-aminopropan, 1-Amino-3-(di-i-undecyl)-aminopropan, 1-Amino-3-(di-n-dodecyl)-aminopropan oder 1-Amino-3-(di-i-dodecyl)-aminopropan.

Die vorstehend beschriebenen 1-Amino-3-dialkylaminopropane sind leicht aus N,N-(dialkyl)aminen und Acrylnitril zugänglich (vgl. Ullmanns Encyclopedia of Industrial Chemistry, Vol. A2, 1985).

Schließlich zählen zu den Diaminen, die geeignete Kationen für die erfindungsgemäßen, nichtwäßrigen ionischen Flüssigkeiten ergeben, auch heterocyclische Verbindungen. Zu ihnen zählen gesättigte oder ungesättigte sowie aromatische Verbindungen mit jeweils zwei dreibindigen N-Atomen im 4- bis 10-, vorzugsweise 5- oder 6-gliedrigen heterocyclischen Ring. Diese Verbindungen können sowohl an den Kohlenstoffatomen als auch an den Stickstoffatomen substituiert sein, vorzugsweise durch Alkylreste mit 1 bis 10 Kohlenstoffatomen und durch Phenylreste. Sie können weiterhin durch, gegebenenfalls substituierte, Benzolringe und/oder Cyclohexanringe unter Ausbildung mehrkerniger Strukturen anelliert sein. Beispiele für solche Verbindungen sind Pyrazol, 3,5-Dimethylpyrazol, Imidazol, Benzimidazol, Dihydropyrazol, Pyrazolidin, Pyridazin, Pyrimidin, Pyrazin, 2,3-, 2,5- und 2,6-Dimethylpyrazin, Cimolin, Phthalazin, Chinazolin, Phenazin und Piperazin. Insbesondere vom Imidazol und seinen Alkyl- und Phenylderivaten abgeleitete Kationen der allgemeinen Formel (12) 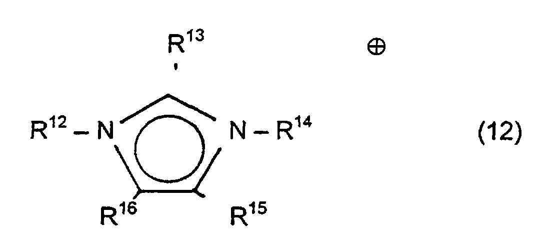 haben sich als Bestandteil der neuen ionischen Flüssigkeiten bewährt. In dieser Formel sind R¹², R¹³, R¹⁴, R¹⁵ und R¹⁶ gleich oder verschieden. Sie stehen für Wasserstoff, einen C₁- bis C₃₀-Alkylrest, einen C₆- bis C₄₀-Arylrest, einen C₇- bis C₄₀-Alkylarylrest oder einen SiR₃¹⁷-rest, in dem R¹⁷ einen C₁bis C₃₀-Alkylrest oder einen C₆- bis C₄₀-Arylrest bedeutet. Beispiele für solche Kationen sind: 1-Ethyl-3-methyl-2,4,5-H-imidazolium, 1-Propyl-3-methyl-2,4,5-H-imidazolium, 1-Butyl-3-methyl-2,4,5-H-imidazolium, 1,3,4,5-Tetramethyl-2-H-imidazolium, 2,4,5-Trimethyl-1,3-H-imidazolium, 1,2,3,4,5-Pentamethylimidazolium, 1,2,3,5-Tetramethyl-4-H-imidazolium, 1,2,3,4-Tetramethyl-5-H-imidazolium, 1,3,4,5-Tetraphenyl-2-H-imidazolium, 1,3-Dimethyl-4,5-diphenyl-2-H-imidazolium, 1-Ethyl-3-isopropyl-2,4,5-H-imidazolium, 1-Butyl-3-octanyl-2,4,5-H-imidazolium, 1-Propyl-3-octanyl-2,4,5-H-imidazolium, 1-Ethyl-3-octanyl-2,4,5-H-imidazolium, 1-Methyl-3-octanyl-2,4,5-H-imidazolium, 1,3-Diisoproypl-4,5-dimethyl-2-H-imidazolium, 1,4,5-Trimethyl-3-trimethylsilyl-2-H-imidazolium, 2-Ethyl-4-methyl-1,3,5-H-imidazolium, 1,3-Adamantyl-4,5-dimethyl-1-H-imidazolium, 1,2,4,5-Tetramethyl-3-H-imidazolium, 1-Methyl-2,3,4,5-H-imidazolium, 1,3-Dimethyl-2,4,5-H-imidazolium, 2-Methyl-4,5-ethyl-1,3-H-imidazolium, 2,4,5-Trimethyl-1,3-H-imidazolium, 1-Ethyl-2,3,4,5-H-imidazolium, 1,3-Diethyl-4,5-dimethyl-2-H-imidazolium, 1,3-Diphenyl-4,5-dimethyl-2-H-imidazolium, 1,3-Diphenyl-2,4,5-H-imidazolium, 1,3-Dimethoxy-4,5-dimethyl-2-H-imidazolium, 1-Trimethylsilyl-2,3,5-trimethyl-4-H-imidazolium.

Weiterhin haben sich ionische Flüssigkeiten auf Basis sulfonierter oder carboxylierter Triester der phosphorigen Säure sehr bewährt, deren Kationen sich von Polyaminen ableiten. Beispiele für solche Polyamine sind Hexamethylentetramin und Purin sowie dessen Derivate.

Zur Herstellung der erfindungsgemäßen nichtwäßrigen ionischen Flüssigkeiten geht man von Salzen der weiter oben ausführlich beschriebenen sulfonierten oder carboxylierten Ester der phosphorigen Säure aus. Geeignet sind Salze der Alkali- und Erdalkalimetalle, vorzugsweise die Natrium- oder Kaliumsalze. Sie werden als wäßrige Lösungen der reinen Verbindungen oder aber auch als Mischung verschiedener Salze eingesetzt.

Zur Gewinnung der erfindungsgemäßen nichtwäßrigen ionischen Flüssigkeiten wird das Amin zu dem einfach- oder mehrfachgeladenen Kation mit Säuren und/oder Alkylierungsmitteln in Gegenwart einer wäßrigen Lösung von Salzen der sulfonierten oder carboxylierten Phosphorigsäureester protoniert oder alkyliert.

Als Säuren können Wasserstoffsäuren, z.B. Tetrafluoroborsäure oder Hexafluorophosphorsäure oder Sauerstoffsäuren, z.B. Phosphorsäure, Schwefelsäure, Salpetersäure, femer Phosphonsäuren mit 1 bis 20 Kohlenstorfatomen oder Sulfonsäuren mit 1 bis 20 Kohlenstoffatomen verwendet werden. Vorzugsweise setzt man wäßrige Schwefelsäure- oder Phosphorsäurelösungen ein, die im allgemeinen 10 bis 30 Gew.-%ige Säure enthalten.

Als Alkylierungsmittel verwendet man z.B. Mono- oder Dialkylsulfate oder Dialkylcarbonate mit 1 bis 41 Kohlenstoffatomen oder Alkylhalogenide mit 1 bis 10 Kohlenstoffatomen.

Säure und/oder Alkylierungsmittel setzt man üblicherweise in einer Menge von 0,9 bis 2,0, vorzugsweise 1,0 bis 1,5 Äquivalenten je Äquivalent der verwendeten Amine zu. Bei Verwendung einer Säure beträgt der pH-Wert nach Säurezugabe 2 bis 5, vorzugsweise 3 bis 4.

Zur Substitution der Metallionen in den Salzen der Phosphorigsäure-Ester durch Ammoniumionen setzt man die Amine, bezogen auf die Metallionen, vorteilhaft im Überschuß über die stöchiometrisch erforderliche Menge ein. Dieser Überschuß beträgt im allgemeinen bis zu 5 Äquivalenten, vorzugsweise bis zu 1 Äquivalent.

Das Amin wird üblicherweise als 20 bis 70 Gew.-%ige, vorzugsweise 40 bis 60 Gew.-%ige Lösung in einem organischen Lösungsmittel verwendet. Als organische Lösungsmittel sind aliphatische oder aromatische Kohlenwasserstoffe, wie Benzol, Toluol, o-Xylol, m-Xylol, p-Xylol, Mesitylen, n-Heptan, n-Octan, Cyclohexan oder auch Ether, z.B. 1,4-Dioxan oder Tetrahydrofuran geeignet. Vorzugsweise verwendet man Toluol oder Cyclohexan.

Die Zugabe der Säure und/oder des Alkylierungsmittels zu der Mischung aus der wäßrigen Lösung des oder der Salze des Phosphorigsäure-Esters und der organischen Lösung des Amins erfolgt bei 0 bis 60°C, vorzugsweise 20 bis 30°C. Die Dauer der Zugabe liegt im allgemeinen zwischen 0,5 bis 3 Stunden, vorzugsweise zwischen 1 und 2 Stunden.

Als Ergebnis der Umsetzung erhält man drei Phasen, eine untere wäßrige Phase, die das aus den Estern der phosphorigen Säure freigesetzte Alkaliund/oder Erdalkalisalz gelöst enthält, eine mittlere Phase, nämlich die nichtwäßrige ionische Flüssigkeit und eine obere Phase, die aus dem organischen Lösungsmittel, das gegebenenfalls überschüssiges Amin enthält, besteht. Die gewünschte nichtwäßrige ionische Flüssigkeit läßt sich durch einfache Phasentrennung gewinnen.

Um eine einwandfreie Ausbildung der drei Phasen sicherzustellen, kann es sich als zweckmäßig erweisen, nach Zugabe der Säure und/oder des Alkylierungsmittels dem Gemisch weiteres organisches Lösungsmittel hinzuzusetzen. Vorzugsweise verwendet man das gleiche organische Lösungsmittel wie zum Lösen des Amins. Die Menge des zugesetzten organischen Lösungsmittels, die erforderlich ist, um eine Trennung in drei Phasen zu erreichen, kann durch einfache Vorversuche ermittelt werden.

In einer weiteren Ausführungsform des Herstellungsverfahrens kann zunächst eine wäßrige Lösung von Salzen der Phosphorigsäure-Ester mit einer Säure und/oder mit einem Alkylierungsmittel versetzt und anschließend das Amin, gelöst in einem organischen Lösungsmittel, zugegeben werden. Es ist auch möglich, das zu protonierende und/oder zu alkylierende Amin zunächst mit der Säure und/oder dem Alkylierungsmittel umzusetzen und anschließend eine wäßrige Lösung der Salze der sulfonierten oder carboxylierten Ester der phosphorigen Säure hinzuzugeben.

Schließlich ist es auch möglich, die Salze der sulfonierten oder carboxylierten Phosphorigsäure-Ester durch Behandlung mit einem Kationenaustauscher in der H⁺-Form in die freie Sulfonsäure bzw. Carbonsäure zu überführen und diese Säure daraufhin mit dem Amin zu neutralisieren.

Die erfindungsgemäßen nichtwäßrigen ionischen Flüssigkeiten eignen sich, wie andere ionische Flüssigkeiten, hervorragend als inerte, thermisch stabile Reaktionsmedien. Durch Variation des chemischen Aufbaus der ionischen Phosphorigsäure-Ester sowohl im kationischen als auch im anionischen Teil des Moleküls wie auch durch Mischung unterschiedlicher Ester oder Salze können Flüssigkeiten entwickelt werden, die speziellen Anforderungen Rechnung tragen. So lassen sich Medien erzeugen, in denen Ausgangsstoffe und Reaktionsprodukte und, sofern es sich um eine katalytische Reaktion handelt, auch die Katalysatoren löslich sind. Gegebenenfalls kann die Löslichkeit eines oder mehrerer Reaktionspartner oder auch des oder der Reaktionsprodukte durch Zusatz bekannter Lösungsvermittler zur ionischen Flüssigkeit verbessert werden. Die Reaktion läuft dann in homogener Phase ab. Eine Abwandlung des homogenen Reaktionsablaufs kann darin bestehen, daß das Umsetzungsprodukt im Reaktionsmedium nicht löslich ist, sich aus dem Reaktionsgemisch abscheidet und daher in einfacher Weise vom Reaktionsgemisch abgetrennt werden kann. Schließlich lassen sich durch geeignete Auswahl der Molekülbestandteile der erfindungsgemäßen ionischen Flüssigkeit oder ihrer Mischungen auch Reaktionsmedien bereitstellen, die, im Falle katalytischer Umsetzungen, lediglich als Lösungsmittel für den Katalysator wirken, die Reaktionspartner also an der Phasengrenzfläche zur Katalysatorlösung miteinander reagieren und das Reaktionsprodukt eine separate Phase bildet. Diese Reaktionsform ermöglicht einen unproblematischen Wiedereinsatz des Katalysators und vereinfacht darüber hinaus seine Regenerierung und Wiederaufarbeitung.

Als Lösungsmittel für Katalysatoren ist in diesem Zusammenhang von besonderer Bedeutung, daß die neuen ionischen Flüssigkeiten auf Grund des Vorhandenseins von dreibindigem Phosphor im Molekül gegenüber den Übergangsmetallen als Liganden wirken und mit ihnen katalytisch wirksame Komplexverbindungen bilden können, so daß sie selbst Komponente eines Katalysatorsystems sind. In dieser Funktion, die als "Ligandflüssigkeit" bezeichnet werden kann, eingesetzt, erlauben sie es, hohe molare Verhältnisse von Ligand zu Übergangsmetall, die 100 und mehr betragen können und bisher nicht realisierbar waren, einzustellen. Solche hohen Ligandüberschüsse sind vielfach erwünscht, denn sie stabilisieren die katalytisch wirksamen Metallkomplexverbindungen und führen auf diese Weise zu einer erheblichen Steigerung der Katalysatorlebensdauer und zur Aufrechterhaltung einer konstanten Katalysatorselektivität über einen langen Zeitraum.

Als Lösungsmittel und als Katalysatorkomponenten können die neuen ionischen Flüssigkeiten für Katalysatoren auf Basis von Übergangsmetallen der Gruppen VI, VII und VIII des Periodensystems der Elemente eingesetzt werden. Besonders geeignet als Katalysatormetalle sind Kobalt, Rhodium, Iridium, Ruthenium, Palladium oder Platin. Zur Herstellung der Katalysatoren gelangen die Übergangsmetalle in elementarer Form, als Metall oder als Verbindung zum Einsatz. In metallischer Form verwendet man sie entweder als feinverteilte Partikel oder in dünner Schicht auf einem Träger wie Aktivkohle, Calciumcarbonat, Aluminiumsilikat oder Tonerde niedergeschlagen. Als Verbindungen finden z.B. Metalloxide oder Salze anorganischer Wasserstoff- und Sauerstoffsäuren, wie Chloride, Nitrate, Sulfate oder Phosphate, femer Carbonylverbindungen, Komplexverbindungen, wie Cyclooctadienylkomplexe, Cyclopentadienylkomplexe, Acetylacetonatokomplexe oder Salze aliphatischer Mono- und Polycarbonsäuren, wie Acetate, Propionate, Butyrate, Valerate, 2-Ethylhexanoate, Oxalate oder Malonate Anwendung. Vorzugsweise verwendet man die 2-Ethylhexanoate.

Die für den jeweiligen Zweck geeigneten Katalysatorsysteme können zunächst in einem Präformierungsschritt gebildet und dann dem Reaktionsgemisch zugesetzt werden. Dabei wird zu der nichtwäßrigen ionischen Ligandflüssigkeit die gewünschte Menge des Übergangsmetalls, entweder in metallischer Form oder als Verbindung, hinzugegeben. Darauf führt man die Reaktanten zu und setzt die Reaktionspartner in Gegenwart des Katalysators um.

Mit gleich gutem Erfolg läßt sich das Katalysatorsystem auch unter Reaktionsbedingungen, also in Gegenwart der umzusetzenden Ausgangsstoffe herstellen.

Die Umsetzungen können sowohl absatzweise als auch kontinuierlich durchgeführt werden. Nach beendeter Umsetzung liegen die Wertprodukte und das Katalysatorsystem in separaten Phasen vor, die durch einfache Trennnung voneinander geschieden werden können. Nach erfolgter Phasentrennung läßt sich das Katalysatorsystem wieder in den Umsetzungsprozeß zurückführen.

Durch die Verwendung der erfindungsgemäßen nichtwäßrigen ionogenen Ligandflüssigkeiten in chemischen Prozessen, die durch Übergangsmetalle katalysiert werden, kann auf die Zugabe zusätzlicher, nicht als Liganden dienender Anionen verzichtet werden.

Das folgende Beispiel soll die Erfindung erläutern, diese aber nicht einschränken. Das in dem Beispiel eingesetzte Phosphit wurde analog Literaturangaben (EP 353770) hergestellt.

### Beispiel :

In einem 1l Dreihalskolben mit Bodenablauf wurden bei Raumtemperatur 103,9g einer 4 Natriumsulfonat-phenyl-(3,3' di-t-butyl-5,5'-dimethoxy-1,1'-biphenyl-2,2'-diyl)phosphit (200 mmol) gelöst in 396g dest. Wasser mit einer Lösung von 65,3g 1-Amino-3-(di-i-nonyl)-aminopropan (200 mmol) in 400 ml Toluol langsam versetzt. Unter Rühren wird über einen Zeitraum von 2 Stunden 98g Schwefelsäure (20 Gew%ig) zugetropft. Nach dem Abstellen des Rührers bilden sich 3 Phasen aus, die jeweils abgetrennt und auf ihren P(III)-Gehalt analysiert wurden. Die gesamte P(III) Menge befindet sich in den 280g der mittleren Phase, welche die nichtwäßrige, ionogene Ligandflüssigkeit bildet. Die untere Phase enthält Natriumhydrogensulfat bzw. Natriumsulfat, während die obere Phase hauptsächlich aus Toluol besteht.

## Patentansprüche

1. Nichtwäßrige ionische Flüssigkeiten der allgemeinen Formel (Q⁺)ₐA^{a-}, in der Q⁺ ein einfach geladenes, gegebenenfalls durch organische Reste substituiertes Ammonium-Kation oder das Äquivalent eines mehrfach geladenen, gegebenenfalls durch organische Reste substitutierten Ammonium-Kations ist, A^{a-} für das Anion eines sulfonierten oder carboxylierten Triesters der phosphorigen Säure steht und a eine ganze Zahl und mindestens gleich 1 ist.

2. Nichtwäßrige ionische Flüssigkeiten nach Anspruch 1, **dadurch gekennzeichnet, daß** die Alkoholkomponente des Phosphorigsäuretriesters der allgemeinen Formel
(Qac)_{b}-Y-(OH)_{c} (1)
entspricht, in der Y ein organischer Rest ist, Q ein, gegebenenfalls durch organische Reste substituiertes Ammonium-Kation oder das Äquivalent eines mehrfach geladenen, gegebenenfalls durch organische Reste substituierten Ammonium-Kations bedeutet, ac für einen Sulfonsäure- oder Carbonsäurerest steht und b und c ganze Zahlen sind und jeweils mindestens den Wert 1, c insbesondere den Wert 1 oder 2 haben.

3. Nichtwäßrige ionische Flüssigkeiten nach Anspruch 2, **dadurch gekennzeichnet, daß** Y ein linearer oder verzweigter, gesättigter aliphatischer Rest mit 1 bis 20 Kohlenstoffatomen ist, der durch Hydroxy- oder durch Alkoxyreste mit 1 bis 10 Kohlenstoffatomen substituiert sein kann.

4. Nichtwäßrige ionische Flüssigkeiten nach Anspruch 2, **dadurch gekennzeichnet, daß** Y ein gesättigter oder ungesättigter, ein- oder mehrkemiger cycloaliphatischer Rest mit 5 bis 14 Kohlenstoffatomen im Ring oder den Ringen, der gegebenenfalls durch Alkylreste mit 1 bis 20 Kohlenstoffatomen, durch Aryl-, Alkylaryl- oder Aralkylreste mit 6 bis 30 Kohlenstoffatomen, durch Cycloalkylreste mit 5 bis 14 Kohlenstoffatomen, durch Hydroxygruppen und durch Alkoxyreste mit 1 bis 10 Kohlenstoffatomen substituiert sein kann.

5. Nichtwäßrige ionische Flüssigkeiten nach Anspruch 2, **dadurch gekennzeichnet, daß** Y ein ein- oder mehrkerniger aromatischer Rest mit 6 bis 14 Kohlenstoffatomen im Ring oder den Ringen, der gegebenenfalls durch Alkylreste mit 1 bis 20 Kohlenstoffatomen, durch Aryl-, Alkylaryl- oder Aralkylreste mit 6 bis 30 Kohlenstoffatomen, durch Cycloalkylreste mit 5 bis 14 Kohlenstoffatomen, durch Hydroxygruppen und durch Alkoxyreste mit 1 bis 10 Kohlenstoffatomen substituiert sein kann.

6. Nichtwäßrige ionische Flüssigkeiten nach Anspruch 5, **dadurch gekennzeichnet, daß** der aromatische Rest sich von Benzol, Toluol, Ethylbenzol, den isomeren Xylolen, von Biphenyl, Naphthalin oder Binaphthyl ableiten und der Aralkylrest ein gegebenenfalls substituierter Benzylrest ist.

7. Nichtwäßrige ionische Flüssigkeiten nach Anspruch 2, **dadurch gekennzeichnet, daß** Y ein gesättigter oder ungesättigter heterocyclischer Rest ist mit einem Heteroatom oder mehreren gleichen oder verschiedenen Heteroatomen aus der Gruppe N, O, S im Molekül und der heterocyclische Rest gegebenenfalls durch Alkylreste oder Arylreste substituiert ist oder mit aliphatischen, cycloaliphatischen oder aromatischen Ringsystemen kondensiert ist.

8. Nichtwäßrige ionische Flüssigkeiten nach Anspruch 7, **dadurch gekennzeichnet, daß** sich der heterocyclische Rest von einer Stickstoff enthaltenden, gesättigten oder ungesättigten Fünfring- oder Sechsringverbindung, insbesondere vom Pyridin, ableitet.

9. Nichtwäßrige ionische Flüssigkeiten nach den Ansprüchen 1 bis 8, **dadurch gekennzeichnet, daß** sie von sulfonierten oder carboxylierten Triestern der phosphorigen Säure abgeleitete Anionen der allgemeinen Formel (2) 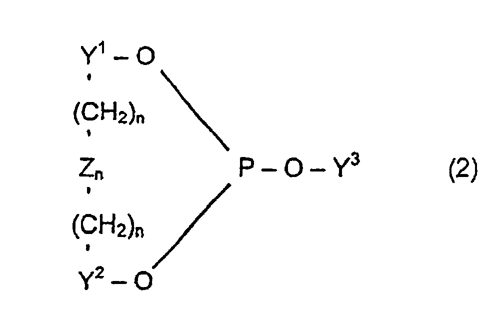 enthalten, in der Y¹, Y² und Y³ gleich oder verschieden sind und einen organischen Rest bedeuten, Z für eine zweiwertige Brückengruppe, nämlich für -CR¹R²-, wobei R¹ und R² unabhängig voneinander Wasserstoff oder Alkylreste mit 1 bis 12 Kohlenstoffatomen sind, für -O-, -S-, -CO-, -CH₂-CO-CH₂- steht und n gleich oder verschieden und 0 oder 1 und im Falle Z gleich -CR¹R²- 1, 2 oder 3 ist und der Ester gemäß Formel (2) mindestens eine Sulfonsäuregruppe oder eine Carboxylgruppe enthält.

10. Nichtwäßrige ionische Flüssigkeiten nach Anspruch 9, **dadurch gekennzeichnet, daß** Y¹, Y² und Y³ gleich oder verschieden sind und einen vom Benzol, vorn Napthalin, vom Biphenyl oder vom Binapthyl abgeleiteten Rest bedeuten, der jeweils durch einen oder mehrere Alkylreste mit 1 bis 20 Kohlenstoffatomen, durch einen oder mehrere Aryl-, Aralkyl-, Alkylarylreste mit 6 bis 30 Kohlenstoffatomen und/oder durch einen oder mehrere Cycloalkylreste mit 5 bis 14 Kohlenstoffatomen, durch Hydroxygruppen und/oder durch Alkoxyreste mit 1 bis 10 Kohlenstoffatomen substituiert sein kann und Z für den Rest -CH₂-, für -O-, -CO- und -CH₂-CO-CH₂- steht.

11. Nichtwäßrige ionische Flüssigkeiten nach den Ansprüchen 1 bis 8, **dadurch gekennzeichnet, daß** sie von sulfonierten oder carboxylierten Triestem der phosphorigen Säure abgeleitete Anionen der allgemeinen Formel (3) 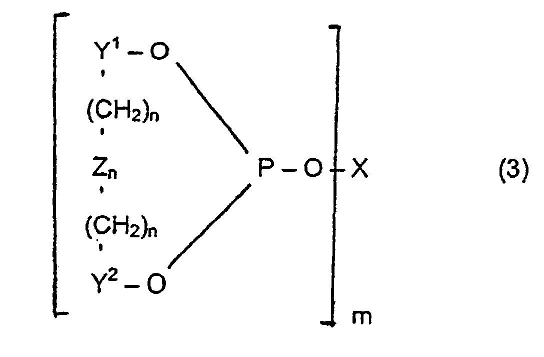 enthalten, in der Y¹ und Y² gleich oder verschieden sind und einen organischen Rest bedeuten, Z für eine zweiwertige Brückengruppen, nämlich für -CR¹R²-, wobei R¹ und R² unabhängig voneinander Wasserstoff oder Alkylreste mit 1 bis 12 Kohlenstoffatomen sind, für -O-, -S-, -CO-, -CH₂-CO-CH₂-, steht, n gleich oder verschieden ist und 0 oder 1 entspricht und im Falle Z gleich -CR¹R²- 1, 2 oder 3 ist, X eine m-werfige Brückengruppe aus der Gruppe der Alkylenreste, Alkylenoxyalkylenreste, Arylenreste oder Aryl-Zₙ-arylreste bedeutet, m eine ganze Zahl von 2 bis 6 ist und der Ester gemäß Formel (3) mindestens eine Sulfonsäuregruppe oder eine Carboxylgruppe enthält.

12. Nichtwäßrige ionische Flüssigkeiten nach Anspruch 11, **dadurch gekennzeichnet, daß** Y¹ und Y² gleich oder verschieden sind und einen vom Benzol, vom Naphthalin, vom Biphenyl oder vom Binaphthyl abgeleiteten Rest bedeuten, der jeweils durch einen oder mehrere Alkylreste mit 1 bis 20 Kohlenstoffatomen, durch einen oder mehrere Aryl-, Aralkyl-, Alkylarylreste mit 6 bis 30 Kohlenstoffatomen und/oder durch einen oder mehrere Cycloalkylreste mit 5 bis 14 Kohlenstoffatomen, durch Hydroxygruppen oder durch Alkoxyreste mit 1 bis 10 Kohlenstoffatomen substituiert sein kann, Z für den Rest -CH₂- für -O-, -COund -CH₂-CO-CH₂- steht und X ein Alkylenrest mit 2 bis 18, insbesondere 2 bis 12, Kohlenstoffatomen ist und in der Bedeutung Aryl-Zₙ-aryl Z für -CH₂-, -O-, -CO- und -CH₂-CO-CH₂- steht, wobei die durch X bezeichneten Reste durch einen oder mehrere Alkyl- und/oder Alkoxyreste und/oder durch einen oder mehrere -SO₃⁻- und/oder -COO⁻-Gruppen substituiert sein können.

13. Nichtwäßrige ionische Flüssigkeiten nach den Ansprüchen 1 bis 8, **dadurch gekennzeichnet, daß** sie von sulfonierten oder carboxylierten Triestern der phosphorigen Säure abgeleitete Anionen der allgemeinen Formel (4) 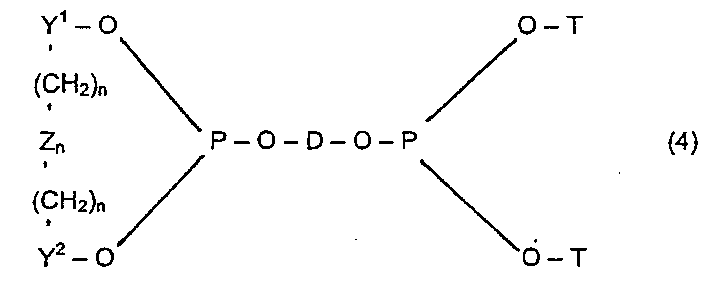 enthalten, in der Y¹ und Y² gleich oder verschieden sind und einen organischen Rest bedeuten, Z für eine zweiwertige Brückengruppe, nämlich für -CR¹R²-, wobei R¹ und R² unabhängig voneinander Wasserstoff oder Alkylreste mit 1 bis 12 Kohlenstoffatomen sind, für -O-, -S-, -CO-, -CH₂-CO-CH₂- steht, n gleich oder verschieden ist und 0 oder 1 entspricht und im Falle -CR¹R²- 1, 2 oder 3 ist, D einen zweiwertigen Kohlenwasserstoffrest als Brückengruppe, nämlich einen Alkylenrest mit 1 bis 30 Kohlenstoffatomen, einen Arylen-, Alkylarylen-, Arylalkylenrest mit 6 bis 30 Kohlenstoffatomen und einen Aryl-Zₙ-aryl-Rest bedeutet und T einen einwertigen Kohlenwasserstoffrest mit 1 bis 30 Kohlenstoffatomen wiedergibt und der Ester gemäß Formel (4) mindestens eine Sulfonsäuregruppe oder eine Carboxylgruppe enthält.

14. Nichtwäßrige ionische Flüssigkeiten nach Anspruch 13, **dadurch gekennzeichnet, daß** Y¹ und Y² gleich oder verschieden sind und einen vom Benzol, vom Napthalin, vom Biphenyl oder vom Binaphthyl abgeleiteten Rest bedeuten, der jeweils durch einen oder mehrere Alkylreste mit 1 bis 20 Kohlenstoffatomen, durch einen oder mehrere Aryl-, Aralkyl-, Alkylarylreste mit 6 bis 30 Kohlenstoffatomen und/oder durch einen oder mehrere Cycloalkylreste mit 5 bis 14 Kohlenstoffatomen, durch Hydroxygruppen oder durch Alkoxyreste mit 1 bis 10 Kohlenstoffatomen substituiert sein kann und Z für den Rest -CH₂-, für -O-, -CO- und -CH₂-CO-CH₂- steht.

15. Nichtwäßrige ionische Flüssigkeiten nach den Ansprüchen 1 bis 14, **dadurch gekennzeichnet, daß** sie von Monoaminen abgeleitete Ammoniumionen der allgemeinen Formel (5) oder (6)
^{⊕}NR³R⁴R⁵R⁶ (5)
R³R⁴N^{⊕} = CR⁵R⁶ (6)
enthalten, wobei R³, R⁴, R⁵, R⁶ gleich oder verschieden sind und Wasserstoff, insbesondere mit der Maßgabe bedeuten, daß mindestens ein R³, R⁴, R⁵, R⁶ nicht Wasserstoff ist oder für einen linearen oder verzweigten aliphatischen Kohlenwasserstoffrest mit 1 bis 20 Kohlenstoffatomen, einen cycloaliphatischen oder aromatischen Kohlenwasserstoffrest mit 6 bis 20 Kohlenstoffatomen oder einen Alkoxyrest mit 1 bis 10 Kohlenstoffatomen stehen.

16. Nichtwäßrige ionische Flüssigkeiten nach den Ansprüchen 1 bis 14, **dadurch gekennzeichnet, daß** sie Ammoniumionen der allgemeinen Formel (7) oder (8) 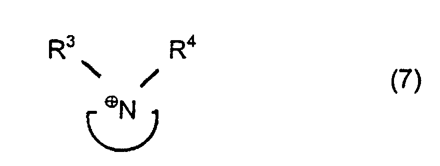 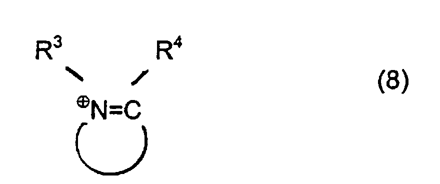 enthalten, die sich von gesättigten oder ungesättigten cyclischen Verbindungen sowie von aromatischen Verbindungen mit jeweils einem dreibindigen N-Atom im 4- bis 10-, vorzugsweise 5- bis 6-gliedrigen heterocyclischen Ring ableiten, wobei R³ und R⁴ gleich oder verschieden sind und Wasserstoff, einen linearen oder verzweigten aliphatischen Kohlenwasserstoffrest mit 1 bis 20 Kohlenstoffatomen, einen cydoaliphatischen oder aromatischen Kohlenwasserstoffrest mit 6 bis 20 Kohlenstoffatomen oder einen Alkoxyrest mit 1 bis 10 Kohlenstoffatomen bedeuten.

17. Nichtwäßrige ionische Flüssigkeiten nach den Ansprüchen 1 bis 14, **dadurch gekennzeichnet, daß** sie von aliphatischen cycloaliphatischen oder aromatischen Diaminen abgeleitete Ammoniumionen der allgemeinen Formel (9) oder (10)
R³R⁴R⁵N^{⊕}-G-N^{⊕}R⁶R⁷R⁸ (9)
R³R⁴N^{⊕} = CR⁵-G-R⁵C = N^{⊕}R³R⁴ (10)
enthalten, wobei R³, R⁴, R⁵, R⁶, R⁷ und R⁸ gleich oder verschieden sind und Wasserstoff, einen linearen oder verzweigten Kohlenwasserstoffrest mit 1 bis 20 Kohlenstoffatomen, einen cycloaliphatischen oder aromatischen Kohlenwasserstoffrest mit 6 bis 30 Kohlenstoffatomen, einen Alkylarylrest mit 7 bis 40 Kohlenstoffatomen oder einen Alkoxyrest mit 1 bis 10 Kohlenstoffatomen bedeuten, G für einen Alkylenrest (-CHR⁹-)_{d}, wobei R⁹ Wasserstoff oder ein Kohlenwasserstoffrest mit 1 bis 5 Kohlenstoffatomen und d eine ganze Zahl von 1 bis 8 ist, für einen Arylenrest mit 6 bis 30 Kohlenstoffatomen oder einen Alkylenarylrest mit 7 bis 40 Kohlenstoffatomen, steht.

18. Nichtwäßrige ionische Flüssigkeiten nach Anspruch 17, **dadurch gekennzeichnet, daß** sie von 1-Amino-3-dialkylaminopropan der allgemeinen Formel (11)
R¹⁰R¹¹N-CH₂-CH₂-CH₂-NH₂ (11)
abgeleitete Ammoniumionen enthalten, wobei R¹⁰ und R¹¹ gleiche oder verschiedene lineare oder verzweigte Alkylreste mit 4 bis 20 Kohlenstoffatomen sind.

19. Nichtwäßrige ionische Flüssigkeiten nach den Ansprüchen 1 bis 14, **dadurch gekennzeichnet, daß** sie von gesättigten, ungesättigten oder aromatischen heterocyclischen Verbindungen mit jeweils zwei dreibindigen N-Atomen im heterocylischen Ring abgeleitete Ammoniumionen enthalten.

20. Nichtwäßrige ionische Flüssigkeiten nach Anspruch 19, **dadurch gekennzeichnet, daß** der heterocyclische Ring 4- bis 10-, vorzugsweise 5-oder 6-gliedrig ist.

21. Nichtwäßrige ionische Flüssigkeiten nach Anspruch 19 oder 20, **dadurch gekennzeichnet, daß** die heterocyclischen Verbindungen an einem oder mehreren Kohlenstoffatomen und/oder an einem oder beiden Stickstoffatomen durch Alkylreste mit 1 bis 10 Kohlenstoffatomen und/oder durch Phenylreste substituiert sind.

22. Nichtwäßrige ionische Flüssigkeiten nach Anspruch 19 oder 20, **dadurch gekennzeichnet, daß** die heterocyclische Verbindung ein, gegebenenfalls ein- oder mehrfach substituiertes, Imidazol ist.

23. Nichtwäßrige ionische Flüssigkeiten nach den Ansprüchen 1 bis 14, **dadurch gekennzeichnet, daß** sie von Polyaminen abgeleitete Ammoniumionen enthalten.

24. Verfahren zur Herstellung nichtwäßriger ionischer Flüssigkeiten nach den Ansprüchen 1 bis 14, **dadurch gekennzeichnet, daß** man eine wäßrige Lösung von Salzen sulfonierter oder carboxylierter Phosphorigsäure-Ester durch Behandlung mit einem Kationenaustauscher in der H⁺-Form in die freie Sulfonsäure bzw. Carbonsäure überführt und die Säure daraufhin mit dem Amin neutralisiert.

25. Verfahren zur Herstellung nichtwäßriger ionischer Flüssigkeiten nach den Ansprüchen 1 bis 14, **dadurch gekennzeichnet, daß** man eine Lösung des das Kation bildende Amins in Gegenwart einer wäßrigen Lösung von Alkali- und/oder Erdalkalisalzen der sulfonierten oder carboxylierten Phosphorigsäure-Ester mit einer Säure und/oder einem Alkylierungsmittel umsetzt.

26. Verfahren zur Herstellung nichtwäßriger ionischer Flüssigkeiten nach Anspruch 25, **dadurch gekennzeichnet, daß** je Äquivalent Amin 0,9 bis 2,0, insbesondere 1,0 bis 1,5 Äquivalente Säure und/oder Alkylierungsmittel verwendet werden.

27. Verfahren zur Herstellung nichtwäßriger ionischer Flüssigkeiten nach den Ansprüchen 25 oder 26, **dadurch gekennzeichnet, daß** als Säure Phosphorsäure, Schwefelsäure, Salpetersäure, Phosphorsäuren mit 1 bis 20 Kohlenstoffatomen oder Sulfonsäuren mit 1 bis 20 Kohlenstoffatomen verwendet werden.

28. Verfahren zur Herstellung nichtwäßriger ionischer Flüssigkeiten nach den Ansprüchen 25 bis 27, **dadurch gekennzeichnet, daß** als Säure eine wäßrige Lösung von Phosphorsäure oder Schwefelsäure verwendet wird.

29. Verfahren zur Herstellung nichtwäßriger ionischer Flüssigkeiten nach den Ansprüchen 25 bis 27, **dadurch gekennzeichnet, daß** als Alkylierungsmittel Mono- oder Dialkylsulfate oder Dialkylcarbonate mit 1 bis 41 Kohlenstoffatomen oder Alkylhalogenide mit 1 bis 10 Kohlenstoffatomen verwendet werden.

30. Verfahren zur Herstellung nichtwäßriger ionischer Flüssigkeiten nach den Ansprüchen 25 bis 29, **dadurch gekennzeichnet, daß** das Amin in Benzol, Toluol, o-Xylol, n-Xylol, p-Xylol, Mesitylen, n-Heptan, n-Octan, Cyclohexan, Tetrahydrofuran oder 1,4-Dioxan gelöst verwendet wird.

31. Verfahren zur Herstellung nichtwäßriger ionischer Flüssigkeiten nach den Ansprüchen 25 bis 30, **dadurch gekennzeichnet, daß** die Umsetzung zwischen Säure und/oder Alkylierungsmittel, Salz des Phosphorigsäure-Esters und Amin bei 0 bis 60°C, vorzugsweise 20 bis 30°C, erfolgt.

32. Verwendung der nichtwäßrigen ionischen Flüssigkeiten nach den Ansprüchen 1 bis 14 als Bestandteil von Katalysatoren auf Basis von Übergangsmetallen.

33. Verwendung der nichtwäßrigen ionischen Flüssigkeiten nach Anspruch 32 als Bestandteil von Katalysatoren in Zweiphasenprozessen.

## Claims

1. A nonaqueous ionic liquid of the formula (Q⁺)ₐA^{a-}, where Q⁺ is a singly charged ammonium cation which may be substituted by organic radicals or the equivalent of a multiply charged ammonium cation which may be substituted by organic radicals, A^{a-} is the anion of a sulfonated or carboxylated triester of phosphorous acid and a is an integer equal to or greater than 1.

2. A nonaqueous ionic liquid as claimed in claim 1, wherein the alcohol component of the phosphorous triester corresponds to the formula
(Qac)_{b}-Y-(OH)_{c} (1)
where Y is an organic radical, Q is an ammonium cation which may be substituted by organic radicals or the equivalent of a multiply charged ammonium cation which may be substituted by organic radicals, ac is a sulfonic acid or carboxylic acid radical and b and c are integers which are each equal to or greater than 1, and c is particularly preferable 1 or 2.

3. A nonaqueous ionic liquid as claimed in claim 2, wherein Y is a linear or branched, saturated aliphatic radical having from 1 to 20 carbon atoms which may be substituted by hydroxy groups or alkoxy radicals having from 1 to 10 carbon atoms.

4. A nonaqueous ionic liquid as claimed in claim 2, wherein Y is a saturated or unsaturated, monocyclic or polycyclic cycloaliphatic radical which has from 5 to 14 carbon atoms in the ring or rings and may be substituted by alkyl radicals having from 1 to 20 carbon atoms, by aryl, alkylaryl or aralkyl radicals having from 6 to 30 carbon atoms, by cycloalkyl radicals having from 5 to 14 carbon atoms, by hydroxy groups or by alkoxy radicals having from 1 to 10 carbon atoms.

5. A nonaqueous ionic liquid as claimed in claim 2, wherein Y is a monocyclic or polycyclic aromatic radical which has from 6 to 14 carbon atoms in the ring or rings and may be substituted by alkyl radicals having from 1 to 20 carbon atoms, by aryl, alkylaryl or aralkyl radicals having from 6 to 30 carbon atoms, by cycloalkyl radicals having from 5 to 14 carbon atoms, by hydroxy groups or by alkoxy radicals having from 1 to 10 carbon atoms.

6. A nonaqueous ionic liquid as claimed in claim 5, wherein the aromatic radical is derived from. benzene, toluene, ethylbenzene, the isomeric xylenes, from biphenyl, naphthalene or binaphthyl and the aralkyl radical is a substituted or unsubstituted benzyl radical.

7. A nonaqueous ionic liquid as claimed in claim 2, wherein Y is a saturated or unsaturated heterocyclic radical which contains a hetero atom or a plurality of identical or different hetero atoms selected from the group consisting of N, O, S in the molecule and may be substituted by alkyl radicals or aryl radicals or be fused with aliphatic, cycloaliphatic or aromatic ring systems.

8. A nonaqueous ionic liquid as claimed in claim 7, wherein the heterocyclic radical is derived from a nitrogen-containing, saturated or unsaturated five-membered or six-membered cyclic compound, in particular from pyridine.

9. A nonaqueous ionic liquid as claimed in any of claims 1 to 8 comprising anions derived from sulfonated or carboxylated triesters of phosphorous acid having the formula (2) 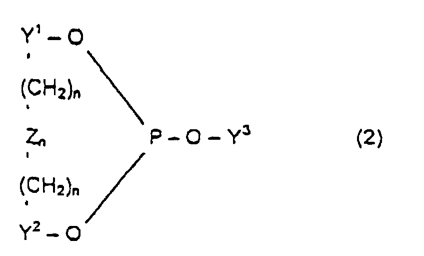 where Y¹, Y² and Y³ are identical or different and are each an organic radical, Z is a divalent bridging group, namely -CR¹R²-, where R¹ and R² are, independently of one another, hydrogen or alkyl radicals having from 1 to 12 carbon atoms, -O-, -S-, -CO-, -CH₂-CO-CH₂-, and n are identical or different and are each 0 or 1 and when Z is -CR¹R²- are 1, 2 or 3 and the ester of the formula (2) contains at least one sulfonic acid group or carboxyl group.

10. A nonaqueous ionic liquid as claimed in claim 9, wherein Y¹, Y² and Y³ are identical or different and are each a radical which is derived from benzene, from naphthalene, from biphenyl or from binaphthyl and may be substituted by one or more alkyl radicals having from 1 to 20 carbon atoms, by one or more aryl, aralkyl, alkylaryl radicals having from 6 to 30 carbon atoms and/or by one or more cycloalkyl radicals having from 5 to 14 carbon atoms, by hydroxy groups and/or by alkoxy radicals having from 1 to 10 carbon atoms, and Z is -CH₂-, -O-, -CO- or -CH₂-CO-CH₂-.

11. A nonaqueous ionic liquid as claimed in any of claims 1 to 8 comprising anions derived from sulfonated or carboxylated triesters of phosphorous acid having the formula (3) 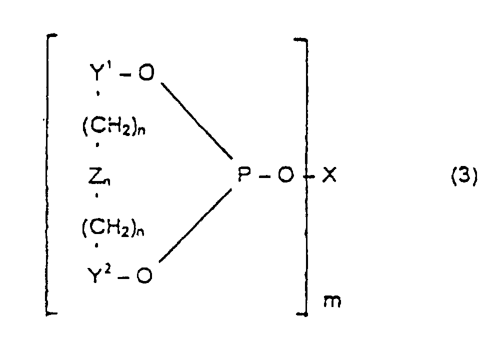 where Y¹ and Y² are identical or different and are each an organic radical, Z is a divalent bridging group, namely -CR¹R²-, where R¹ and R² are, independently of one another, hydrogen or alkyl radicals having from 1 to 12 carbon atoms, -O-, -S-, -CO-, -CH₂-CO-CH₂-, n are identical or different and are each 0 or 1 and when Z is -CR¹R²- are 1, 2 or 3, X is an m-valent bridging group selected from among alkylene radicals, alkyleneoxyalkylene radicals, arylene radicals and aryl-zₙ-aryl radicals, m is an integer from 2 to 6 and the ester of the formula (3) contains at least one sulfonic acid group or carboxyl group.

12. A nonaqueous ionic liquid as claimed in claim 11, wherein Y¹ and Y² are identical or different and are each a radical which is derived from benzene, from naphthalene, from biphenyl or from binaphthyl and may be substituted by one or more alkyl radicals having from 1 to 20 carbon atoms, by one or more aryl, aralkyl, alkylaryl radicals having from 6 to 30 carbon atoms and/or by one or more cycloalkyl radicals having from 5 to 14 carbon atoms, by hydroxy groups or by alkoxy radicals having from 1 to 10 carbon atoms, Z is -CH₂-, -O-, -CO- or -CH₂-CO-CH₂- and X is an alkylene radical having from 2 to 18, in particular from 2 to 12, carbon atoms and when X is aryl-Zₙ-aryl, Z is -CH₂-, -O-, -CO- or -CH₂-CO-CH₂-, where the radicals X may be substituted by one or more alkyl and/or alkoxy radicals and/or by one or more -SO₃⁻ and/or -COO⁻ groups.

13. A nonaqueous ionic liquid as claimed in any of claims 1 to 8 comprising anions derived from sulfonated or carboxylated triesters of phosphorous acid having the formula (4) 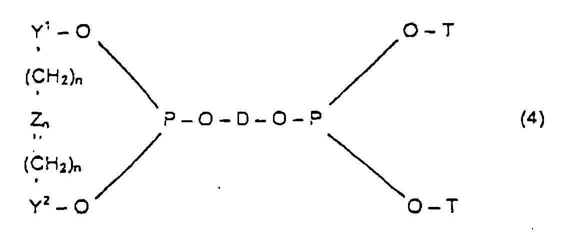 where Y¹ and Y² are identical or different and are each an organic radical, Z is a divalent bridging group, namely -CR¹R²-, where R¹ and R² are, independently of one another, hydrogen or alkyl radicals having from 1 to 12 carbon atoms, -O-, -S-, -CO-, -CH₂-CO-CH₂-, n are identical or different and are each 0 or 1 and when Z is -CR¹R²- are 1, 2 or 3, D is a divalent hydrocarbon radical as bridging group, namely an alkylene radical having from 1 to 30 carbon atoms, an arylene, alkylarylene, arylalkylene radical having from 6 to 30 carbon atoms or an aryl-Zₙ-aryl radical and T is a monovalent hydrocarbon radical having from 1 to 30 carbon atoms, and the ester of the formula (4) contains at least one sulfonic acid group or carboxyl group.

14. A nonaqueous ionic liquid as claimed in claim 13, wherein Y¹ and Y² are identical or different and are each a radical which is derived from benzene, from naphthalene, from biphenyl or from binaphthyl and may be substituted by one or more alkyl radicals having from 1 to 20 carbon atoms, by one or more aryl, aralkyl, alkylaryl radicals having from 6 to 30 carbon atoms and/or by one or more cycloalkyl radicals having from 5 to 14 carbon atoms, by hydroxy groups or by alkoxy radicals having from 1 to 10 carbon atoms and Z is -CH₂-, -O-, -CO- or -CH₂-CO-CH₂-.

15. A nonaqueous ionic liquid as claimed in any of claims 1 to 14 comprising ammonium ions derived from monoamines and having the formula (5) or (6)
^{⊕}NR³R⁴R⁵R⁶ (5)
R³R⁴N^{⊕} = CR⁵R⁶ (6)
where R³, R⁴, R⁵, R⁶ are identical or different and are each hydrogen, in particular with the proviso that at least one of R³, R⁴, R⁵, R⁶ is not hydrogen, or a linear or branched, aliphatic hydrocarbon radical having from 1 to 20 carbon atoms, a cycloaliphatic or aromatic hydrocarbon radical having from 6 to 20 carbon atoms or an alkoxy radical having from 1 to 10 carbon atoms.

16. A nonaqueous ionic liquid as claimed in any of claims 1 to 14 comprising ammonium ions of the formula (7) or (8) 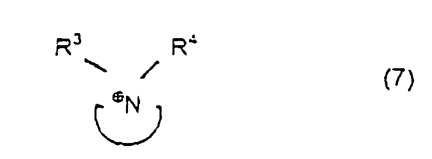 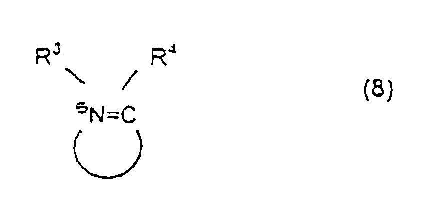 which are derived from saturated or unsaturated cyclic compounds or from aromatic compounds having a trivalent N atom in a 4- to 10-membered, preferably 5-or 6-membered, heterocyclic ring, where R³ and R⁴ are identical or different and are each hydrogen, a linear or branched aliphatic hydrocarbon radical having from 1 to 20 carbon atoms, a cycloaliphatic or aromatic hydrocarbon radical having from 6 to 20 carbon atoms or an alkoxy radical having from 1 to 10 carbon atoms.

17. A nonaqueous ionic liquid as claimed in any of claims 1 to 14 comprising ammonium ions which are derived from aliphatic, cycloaliphatic or aromatic diamines and have the formula (9) or (10)
R³R⁴R⁵N^{⊕}-G-N^{⊕}R⁶R⁷R⁸ (9)
R³R⁴N^{⊕} = CR⁵-G-R⁵C = N^{⊕}R³R⁴ (10)
where R³, R⁴, R⁵, R⁶, R⁷ and R⁸ are identical or different and are each hydrogen, a linear or branched hydrocarbon radical having from 1 to 20 carbon atoms, a cycloaliphatic or aromatic hydrocarbon radical having from 6 to 30 carbon atoms, an alkylaryl radical having from 7 to 40 carbon atoms or an alkoxy radical having from 1 to 10 carbon atoms, G is an alkylene radical (-CHR⁹-)_{d}, where R⁹ is hydrogen or a hydrocarbon radical having from 1 to 5 carbon atoms and d is an integer from 1 to 8, an arylene radical having from 6 to 30 carbon atoms or an alkylenearyl radical having from 7 to 40 carbon atoms.

18. A nonaqueous ionic liquid as claimed in claim 17 comprising ammonium ions derived from 1-amino-3-dialkylaminopropane of the formula (11)
R¹⁰R¹¹N-CH₂-CH₂-CH₂-NH₂ (11)
where R¹⁰ and R¹¹ are identical or different, linear or branched alkyl radicals having from 4 to 20 carbon atoms.

19. A nonaqueous ionic liquid as claimed in any of claims 1 to 14 comprising ammonium ions derived from saturated, unsaturated or aromatic heterocyclic compounds having at least two trivalent N atoms in the heterocyclic ring.

20. A nonaqueous ionic liquid as claimed in claim 19, wherein the heterocyclic ring is from 4- to 10-membered, preferably 5- or 6-membered.

21. A nonaqueous ionic liquid as claimed in claim 19 or 20, wherein the heterocyclic compounds are substituted on one or more carbon atoms and/or on one or both nitrogen atoms by alkyl radicals having from 1 to 10 carbon atoms and/or by phenyl radicals.

22. A nonaqueous ionic liquid as claimed in claim 19 or 20, wherein the heterocyclic compound is an unsubstituted, monosubstituted or polysubstituted imidazole.

23. A nonaqueous ionic liquid as claimed in any of claims 1 to 14 comprising ammonium ions derived from polyamines.

24. A process for preparing nonaqueous ionic liquids as claimed in any of claims 1 to 14, which comprises converting an aqueous solution of salts of sulfonated or carboxylated phosphorous esters into the free sulfonic acid or carboxylic acid by treatment with a cation exchanger in the H⁺ form and then neutralizing this acid with the amine.

25. A process for preparing nonaqueous ionic liquids as claimed in any of claims 1 to 14, which comprises reacting a solution of the amine forming the cation with an acid and/or an alkylating agent in the presence of an aqueous solution of alkali metal and/or alkaline earth metal salts of the sulfonated or carboxylated phosphorous esters.

26. The process for preparing nonaqueous ionic liquids as claimed in claim 25, wherein from 0.9 to 2.0, in particular from 1.0 to 1.5, equivalents of acid and/or alkylating agent are used per equivalent of amine.

27. The process for preparing nonaqueous ionic liquids as claimed in claim 25 or 26, wherein phosphoric acid, sulfuric acid, nitric acid, a phosphoric acid having from 1 to 20 carbon atoms or a sulfonic acid having from 1 to 20 carbon atoms is used as acid.

28. The process for preparing nonaqueous ionic liquids as claimed in any of claims 25 to 27, wherein an aqueous solution of phosphoric acid or sulfuric acid is used as acid.

29. The process for preparing nonaqueous ionic liquids as claimed in any of claims 25 to 27, wherein a monoalkyl or dialkyl sulfate or a dialkyl carbonate having from 1 to 41 carbon atoms or an alkyl halide having from 1 to 10 carbon atoms is used as alkylating agent.

30. The process for preparing nonaqueous ionic liquids as claimed in any of claims 25 to 29, wherein the amine is used as a solution in benzene, toluene, o-xylene, n-xylene, p-xylene, mesitylene, n-heptane, n-octane, cyclohexane, tetrahydrofuran or 1,4-dioxane.

31. The process for preparing nonaqueous ionic liquids as claimed in any of claims 25 to 30, wherein the reaction between acid and/or alkylating agent, salt of the phosphorous ester and amine is carried out at from 0 to 60°C, preferably from 20 to 30°C.

32. The use of a nonaqueous ionic liquid as claimed in any of claims 1 to 14 as a constituent of catalysts based on transition metals.

33. The use of a nonaqueous ionic liquid as claimed in claim 32 as a constituent of catalysts in two-phase processes.

## Revendications

1. Liquides ioniques non aqueux de formule générale (Q⁺)ₐA^{a-}, dans laquelle Q⁺ représente un cation ammonium à charge unique éventuellement substitué par des restes organiques ou l'équivalent d'un cation ammonium à charge multiple éventuellement substitué par des restes organiques, A^{a-} représente Fanion d'un triester sulfoné ou carboxylé de l'acide phosphoreux et a est un nombre entier et est au moins égal à 1.

2. Liquides ioniques non aqueux selon la revendication 1, **caractérisés en ce que** le constituant alcool du triester de l'acide phosphoreux correspond à la formule générale
(Qac)_{b}-Y-(OH)_{c} (1)
dans laquelle Y est un reste organique, Q représente un cation ammonium éventuellement substitué par des restes organiques ou l'équivalent d'un cation ammonium à charge multiple éventuellement substitué par des restes organiques, ac représente un reste d'acide sulfonique ou d'acide carboxylique et b et c sont des nombres entiers et ont chacun au moins la valeur 1, c étant en particulier égal à 1 ou 2.

3. Liquides ioniques non aqueux selon la revendication 2, **caractérisés en ce que** Y représente un reste aliphatique linéaire ou ramifié saturé de 1 à 20 atomes de carbone pouvant être substitué par des restes hydroxy ou alcoxy de 1 à 10 atomes de carbone.

4. Liquides ioniques non aqueux selon la revendication 2, **caractérisés en ce que** Y est un reste cycloaliphatique saturé ou insaturé, monocyclique ou polycyclique, de 5 à 14 atomes de carbone cycliques, pouvant éventuellement être substitué par des restes alkyle de 1 à 20 atomes de carbone, par des restes aryle, alkylaryle ou aralkyle de 6 à 30 atomes de carbone, par des restes cycloalkyle de 5 à 14 atomes de carbone, par des groupes hydroxy et par des restes alcoxy de 1 à 10 atomes de carbone.

5. Liquides ioniques non aqueux selon la revendication 2, **caractérisés en ce que** Y est un reste aromatique mono- ou polycyclique de 6 à 14 atomes de carbone cycliques, pouvant éventuellement être substitué par des restes alkyle de 1 à 20 atomes de carbone, par des restes aryle, alkylaryle ou aralkyle de 6 à 30 atomes de carbone, par des restes cycloalkyle de 5 à 14 atomes de carbone, par des groupes hydroxy et par des restes alcoxy de 1 à 10 atomes de carbone.

6. Liquides ioniques non aqueux selon la revendication 5, **caractérisés en ce que** le reste aromatique est dérivé du benzène, du toluène, de l'éthylbenzène, des xylènes isomères, du biphényle, du naphtalène ou du binaphtyle, et le reste aralkyle est un reste benzyle éventuellement substitué.

7. Liquides ioniques non aqueux selon la revendication 2, **caractérisés en ce que** Y est un reste hétérocyclique saturé ou insaturé contenant dans la molécule un hétéroatome ou plusieurs hétéroatomes identiques ou différents du groupe constitué par N, O, S, et le reste hétérocyclique est éventuellement substitué par des restes alkyle. ou des restes aryle ou condensé avec des systèmes cycliques aliphatiques, cycloaliphatiques ou aromatiques.

8. Liquides ioniques non aqueux selon la revendication 7, **caractérisés en ce que** le reste hétérocyclique est dérivé d'un composé cyclique azoté saturé ou insaturé de 5 ou 6 chaînons, en particulier de la pyridine.

9. Liquides ioniques non aqueux selon les revendications 1 à 8, **caractérisés en ce qu'**ils contiennent des anions dérivés de triesters sulfonés ou carboxylés de l'acide phosphoreux de formule générale (2) 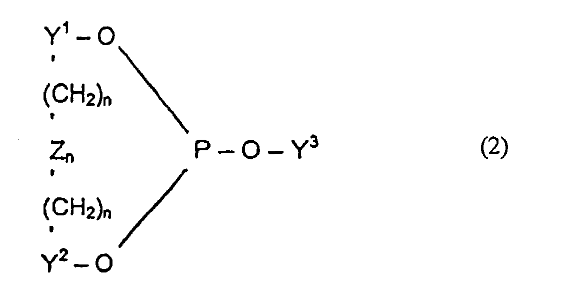 dans laquelle Y¹, Y² et Y³ sont identiques ou différents et représentent un reste organique, Z représente un groupe de pontage divalent, à savoir un groupe -CR¹R²- dans lequel R¹ et R² représentent indépendamment l'un de l'autre l'hydrogène ou des restes alkyle de 1 à 12 atomes de carbone, ou -O-, -S-, -CO-, -CH₂-CO-CH₂-, et les n sont identiques ou différents et sont égaux à 0 ou 1, et, dans le cas où Z est le groupe -CR¹R²-, à 1, 2 ou 3, et l'ester de formule (2) contient au moins un groupe acide sulfonique ou un groupe carboxyle.

10. Liquides ioniques non aqueux selon la revendication 9, **caractérisés en ce que** Y¹, Y² et Y³ sont identiques ou différents et représentent un reste dérivé du benzène, du naphtalène, du biphényle ou du binaphtyle, pouvant à chaque fois être substitué par un ou plusieurs restes alkyle de 1 à 20 atomes de carbone, par un ou plusieurs restes aryle, alkylaryle ou aralkyle de 6 à 30 atomes de carbone et/ou par un ou plusieurs restes cycloalkyle de 5 à 14 atomes de carbone, par des groupes hydroxy et/ou par des restes alcoxy de 1 à 10 atomes de carbone, et Z représente le reste -CH₂-, ou -O-, -CO- et -CH₂-CO-CH₂-.

11. Liquides ioniques non aqueux selon les revendications 1 à 8, **caractérisés en ce qu'**ils contiennent des anions dérivés de triesters sulfonés ou carboxylés de l'acide phosphoreux de formule générale (3) 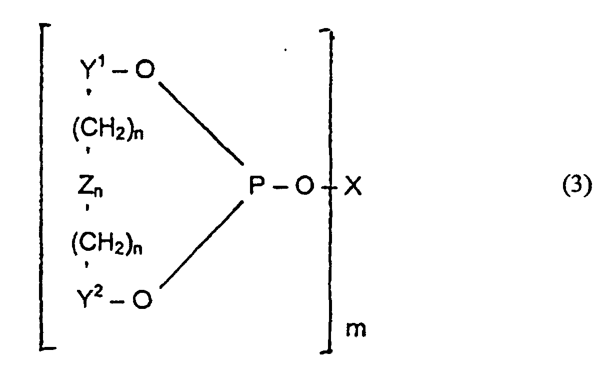 dans laquelle Y¹ et Y² sont identiques ou différents et représentent un reste organique, Z représente un groupe de pontage divalent, à savoir un groupe -CR¹R²- dans lequel R¹ et R² représentent indépendamment l'un de l'autre l'hydrogène ou des restes alkyle de 1 à 12 atomes de carbone, ou -O-, -S-, -CO-, -CH₂-CO-CH₂-, les n sont identiques ou différents et sont égaux à 0 ou 1, et, dans le cas où Z est le groupe -CR¹R²-, à 1, 2 ou 3, X est un groupe de pontage de valence m appartenant au groupe des restes alkylène, des restes alkylène-oxyalkylène, des restes arylène ou des restes aryl-Zₙ-aryle, m est un nombre entier de 2 à 6 et l'ester de formule (3) contient au moins un groupe acide sulfonique ou un groupe carboxyle.

12. Liquides ioniques non aqueux selon la revendication 11, **caractérisés en ce que** Y¹ et Y² sont identiques ou différents et représentent un reste dérivé du benzène, du naphtalène, du biphényle ou du binaphtyle, pouvant à chaque fois être substitué par un ou plusieurs restes alkyle de 1 à 20 atomes de carbone, par un ou plusieurs restes aryle, aralkyle ou alkylaryle de 6 à 30 atomes de carbone et/ou par un ou plusieurs restes cycloalkyle de 5 à 14 atomes de carbone, par des groupes hydroxy ou par des restes alcoxy de 1 à 10 atomes de carbone, Z représente le reste -CH₂-, ou -O-, -CO- et -CH₂-CO-CH₂-, et X est un reste alkylène de 2 à 18, en particulier 2 à 12 atomes de carbone, et, dans la signification aryl-Zₙ-aryle, Z représente -CH₂-, -O-, -CO- et -CH₂-CO-CH₂-, les restes désignés par X pouvant être substitués par un ou plusieurs restes alkyle et/ou alcoxy et/ou par un ou plusieurs groupes -SO₃⁻ et/ou -COO⁻.

13. Liquides ioniques non aqueux selon les revendications 1 à 8, **caractérisés en ce qu'**ils contiennent des anions dérivés de triesters sulfonés ou carboxylés de l'acide phosphoreux de formule générale (4) 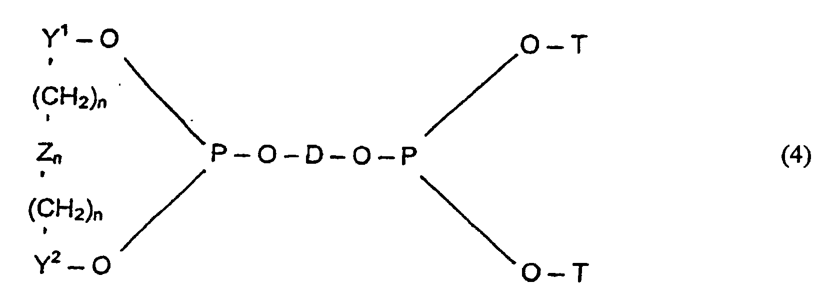 dans laquelle Y¹ et Y² sont identiques ou différents et représentent un reste organique, Z représente un groupe de pontage divalent, à savoir un groupe -CR¹R²- dans lequel R¹ et R² représentent indépendamment l'un de l'autre l'hydrogène ou des restes alkyle de 1 à 12 atomes de carbone, ou -O-, -S-, -CO-, -CH₂-CO-CH₂-, les n sont identiques ou différents et sont égaux à 0 ou 1, et, dans le cas de -CR¹R²-, à 1, 2 ou 3, D est un reste hydrocarboné divalent comme groupe de pontage, à savoir un reste alkylène de 1 à 30 atomes de carbone, un reste arylène, alkylarylène ou arylalkylène de 6 à 30 atomes de carbone et un reste aryl-Zₙ-aryle, et T est un reste hydrocarboné monovalent de 1 à 30 atomes de carbone, et l'ester de formule (4) contient au moins un groupe acide sulfonique ou un groupe carboxyle.

14. Liquides ioniques non aqueux selon la revendication 13, **caractérisés en ce que** Y¹ et Y² sont identiques ou différents et représentent un reste dérivé du benzène, du naphtalène, du biphényle ou du binaphtyle, pouvant à chaque fois être substitué par un ou plusieurs restes alkyle de 1 à 20 atomes de carbone, par un ou plusieurs restes aryle, aralkyle ou alkylaryle de 6 à 30 atomes de carbone et/ou par un ou plusieurs restes cycloalkyle de 5 à 14 atomes de carbone, par des groupes hydroxy ou par des restes alcoxy de 1 à 10 atomes de carbone, et Z représente le reste -CH₂-, ou -O-, -CO- et -CH₂-CO-CH₂-.

15. Liquides ioniques non aqueux selon les revendications 1 à 14, **caractérisés en ce qu'**ils contiennent des ions ammonium dérivés de monoamines de formule générale (5) ou (6)
^{⊕}NR³R⁴R⁵R⁶ (5)
R³R⁴N^{⊕} = CR⁵R⁶ (6)
où R³, R⁴, R⁵ et R⁶ sont identiques ou différents et représentent l'hydrogène, en particulier à condition qu'au moins l'un des R³, R⁴, R⁵ et R⁶ ne soit pas l'hydrogène, ou un reste hydrocarboné aliphatique linéaire ou ramifié de 1 à 20 atomes de carbone, un reste hydrocarboné cycloaliphatique ou aromatique de 6 à 20 atomes de carbone ou un reste alcoxy de 1 à 10 atomes de carbone.

16. Liquides ioniques non aqueux selon les revendications 1 à 14, **caractérisés en ce qu'**ils contiennent des ions ammonium de formule générale (7) ou (8) 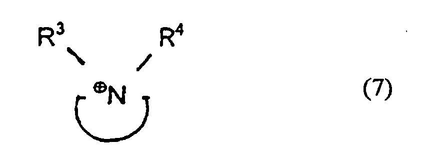 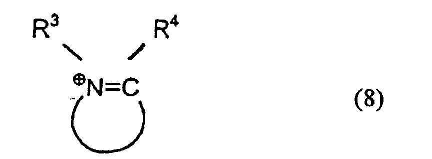 qui dérivent de composés cycliques saturés ou insaturés et de composés aromatiques contenant à chaque fois un atome d'azote à trois liaisons dans l'hétérocycle de 4 à 10, de préférence 5 à 6 chaînons, où R³ et R⁴ sont identiques ou différents et représentent l'hydrogène, un reste hydrocarboné aliphatique linéaire ou ramifié de 1 à 20 atomes de carbone, un reste hydrocarboné cycloaliphatique ou aromatique de 6 à 20 atomes de carbone ou un reste alcoxy de 1 à 10 atomes de carbone.

17. Liquides ioniques non aqueux selon les revendications 1 à 14, **caractérisés en ce que** qu'ils contiennent des ions ammonium dérivés de diamines aliphatiques, cycloaliphatiques ou aromatiques, de formule générale (9) ou (10)
R³R⁴R⁵N^{⊕}-G-N^{⊕}R⁶R⁷R⁸ (9)
R³R⁴N^{⊕} = CR⁵-G-R⁵C = N^{⊕}R³R⁴ (10)
où R³, R⁴, R⁵, R⁶, R⁷ et R⁸ sont identiques ou différents et représentent l'hydrogène, un reste hydrocarboné linéaire ou ramifié de 1 à 20 atomes de carbone, un reste hydrocarboné cycloaliphatique ou aromatique de 6 à 30 atomes de carbone, un reste alkylaryle de 7 à 40 atomes de carbone ou un reste alcoxy de 1 à 10 atomes de carbone, G représente un reste alkylène (-CHR⁹-)_{d} dans lequel R⁹ représente l'hydrogène ou un reste hydrocarboné de 1 à 5 atomes de carbone et d est un nombre entier de 1 à 8, un reste arylène de 6 à 30 atomes de carbone ou un reste alkylène-aryle de 7 à 40 atomes de carbone.

18. Liquides ioniques non aqueux selon la revendication 17, **caractérisés en ce qu'**ils contiennent des ions ammonium dérivés de 1-amino-3-dialkylaminopropane de formule générale (11)
R¹⁰R¹¹N-CH₂-CH₂-CH₂-NH₂ (11)
dans laquelle R¹⁰ et R¹¹ représentent des restes alkyle linéaires ou ramifiés identiques ou différents de 4 à 20 atomes de carbone.

19. Liquides ioniques non aqueux selon les revendications 1 à 14, **caractérisés en ce qu'**ils contiennent des ions ammonium dérivés de composés hétérocycliques saturés, insaturés ou aromatiques contenant à chaque fois deux atomes d'azote à trois liaisons dans l'hétérocycle.

20. Liquides ioniques non aqueux selon la revendication 19, **caractérisés en ce que** l'hétérocycle a 4 à 10, de préférence 5 ou 6 chaînons.

21. Liquides ioniques non aqueux selon la revendication 19 ou 20, **caractérisés en ce que** les composés hétérocycliques sont substitués sur un ou plusieurs atomes de carbone et/ou sur l'un des atomes d'azote ou sur les deux par des restes alkyle de 1 à 10 atomes de carbone et/ou par des restes phényle.

22. Liquides ioniques non aqueux selon la revendication 19 ou 20, **caractérisés en ce que** le composé hétérocyclique est un imidazole éventuellement substitué une ou plusieurs fois.

23. Liquides ioniques non aqueux selon les revendications 1 à 14, **caractérisés en ce qu'**ils contiennent des ions ammonium dérivés de polyamines.

24. Procédé de préparation de liquides ioniques non aqueux selon les revendications 1 à 14, **caractérisé en ce que** l'on transforme une solution aqueuse de sels d'esters sulfonés ou carboxylés de l'acide phosphoreux en l'acide sulfonique libre ou l'acide carboxylique libre par traitement avec un échangeur de cations sous forme H⁺, puis on neutralise l'acide avec l'amine.

25. Procédé de préparation de liquides ioniques non aqueux selon les revendications 1 à 14, **caractérisé en ce que** l'on fait réagir une solution de l'amine formant le cation avec un acide et/ou un agent d'alkylation en présence d'une solution aqueuse de sels de métaux alcalins et/ou de métaux alcalino-terreux des esters sulfonés ou carboxylés de l'acide phosphoreux.

26. Procédé de préparation de liquides ioniques non aqueux selon la revendication 25, **caractérisé en ce que** l'on utilise 0,9 à 2,0, en particulier 1,0 à 1,5 équivalent d'acide et/ou d'agent d'alkylation par équivalent d'aminé.

27. Procédé de préparation de liquides ioniques non aqueux selon les revendications 25 ou 26, **caractérisé en ce que** l'on utilise comme acide l'acide phosphorique, l'acide sulfurique, l'acide nitrique, des acides phosphoriques contenant 1 à 20 atomes de carbone ou des acides sulfoniques contenant 1 à 20 atomes de carbone.

28. Procédé de préparation de liquides ioniques non aqueux selon les revendications 25 à 27, **caractérisé en ce que** l'on utilise comme acide une solution aqueuse d'acide phosphorique ou d'acide sulfurique.

29. Procédé de préparation de liquides ioniques non aqueux selon les revendications 25 à 27, **caractérisé en ce que** l'on utilise comme agent d'alkylation des sulfates de mono- ou dialkyle ou des carbonates de dialkyle de 1 à 41 atomes de carbone ou des halogénures d'alkyle de 1 à 10 atomes de carbone.

30. Procédé de préparation de liquides ioniques non aqueux selon les revendications 25 à 29, **caractérisé en ce que** l'on utilise l'amine en solution dans du benzène, du toluène, de l'o-xylène, du m-xylène, du p-xylène, du mésitylène, du n-heptane, du n-octane, du cyclohexane, du tétrahydrofurane ou du 1,4-dioxane.

31. Procédé de préparation de liquides ioniques non aqueux selon les revendications 25 à 30, **caractérisé en ce que** la réaction entre l'acide et/ou l'agent d'alkylation, le sel de l'ester d'acide phosphoreux et l'amine s'effectue à une température de 0 à 60°C, de préférence de 20 à 30°C.

32. Utilisation des liquides ioniques non aqueux selon les revendications 1 à 14 comme constituants de catalyseurs à base de métaux de transition.

33. Utilisation des liquides ioniques non aqueux selon la revendication 32 comme constituants de catalyseurs dans des procédés à deux phases.
